Europäisches Patentamt

European Patent Office

Office européen des brevets

(19)

(11) **EP 1 241 598 A1**

## (12) EUROPEAN PATENT APPLICATION
published in accordance with Art. 158(3) EPC

(43) Date of publication:
**18.09.2002 Bulletin 2002/38**

(21) Application number: **00987705.1**

(22) Date of filing: **21.12.2000**

(51) Int Cl.⁷: **G06F 17/50**

(86) International application number:
**PCT/JP00/09127**

(87) International publication number:
**WO 01/048640 (05.07.2001 Gazette 2001/27)**

(84) Designated Contracting States:
**AT BE CH CY DE DK ES FI FR GB GR IE IT LI LU MC NL PT SE TR**

(30) Priority: **24.12.1999 JP 36849899**

(71) Applicant: **Kaneka Corporation**
**Osaka-shi, Osaka 530-0005 (JP)**

(72) Inventors:
• **MORIKAWA, Souichi**
  **Himeji-shi, Hyogo 670-0001 (JP)**
• **NAKAI, Takahisa**
  **Kobe-shi, Hyogo 655-0853 (JP)**
• **ISHII, Kiyoto**
  **Akashi-shi, Hyogo 673-0869 (JP)**

(74) Representative: **Joly, Jean-Jacques et al**
  **Cabinet Beau de Loménie**
  **158, rue de l'Université**
  **75340 Paris Cédex 07 (FR)**

(54) **METHOD AND DEVICE FOR CALCULATING OPTIMIZATION SOLUTION OF MULTIPLE MUTANT PROTEIN AMINO ACID SEQUENCE, AND STORAGE MEDIUM WHERE PROGRAM FOR EXECUTING THE METHOD IS STORED**

(57)    The present invention provides a method for calculating an optimized solution of the amino acid sequences of multiple-mutated proteins. The method comprises searching the three-dimensional structural coordinates of amino acid side chains of the amino acid sequences of members of a multiple-mutated protein population based on the three-dimensional structure data of a template protein population using a dead end elimination algorithm, and executing structural energy minimization calculations for the members, thereby calculating the three-dimensional structural coordinates of an optimum multiple-mutated protein, calculating a characteristic value from the three-dimensional structural coordinates of the optimum multiple-mutated protein, and applying a genetic algorithm to the multiple-mutated protein population to calculate the members which optimize the characteristic value. According to the present invention, an optimum solution can be selected from a multiple-mutated protein population having an enormous number of combinations based on a characteristic value without reducing accuracy and within a short time.

*FIG.1*

## EP 1 241 598 A1

**Description**

TECHNICAL FIELD

The present invention relates to a method for calculating an industrially useful optimized solution of the amino acid sequences of multiple-mutated proteins, an apparatus for calculating an optimized solution of the amino acid sequences of multiple-mutated proteins, and a storage medium carrying a program executing a method for calculating an optimized solution of the amino acid sequences of multiple-mutated proteins. More specifically, the present invention relates to a method and apparatus for modifying any or a combination of the thermal stability, chemical stability, chemical selectivity to a substrate, stereoselectivity to a substrate, and optimal pH value of an industrially useful enzyme or signal transduction protein. and a storage medium carrying a program describing such a method. The present invention relates to a computer program for executing calculation of an optimized solution of the amino acid sequences of multiple-mutated proteins and a transmission medium carrying the computer program. The present invention also relates to provision of a service utilizing a method for calculating an optimized solution of the amino acid sequences of multiple-mutated proteins.

BACKGROUND ART

Flexible design and production of proteins having a desired structure and function is one of the major objectives of molecular biology. Conventionally, molecular design of modified proteins has been achieved by modifying the amino acid sequence of a naturally occurring protein by genetic engineering or synthetic chemistry to obtain a modified protein, and analyzing the modified protein by comparing biological functions (e.g., enzyme activity), physicochemical properties (e.g., thermal stability), and the like between the modified protein and the naturally occurring protein.

Some informatic methods for designing a mutated protein having a desired characteristic using a known protein as a template have been developed. Among such methods, design method which directly handle the atomic coordinates of a protein molecule are particularly highly reliable. A representative example of such a method is a method for calculating the atomic coordinates of the amino acid sequences of all multiple-mutated proteins which are candidates for solutions to calculate the characteristics of each mutated protein, for selecting with those results a mutated protein having a desired characteristic.

Now it is considered that a certain candidate for solutions is calculated by such a method. The atomic coordinates of a certain mutated protein molecule are calculated at high speed with good precision by a known calculation method, for example, a dead end elimination method using the high-order structure of a wild-type protein as a template or an optimization method using a dead end elimination algorithm.

(Problems to be Solved by the Invention)

Practically, it is difficult to calculate the atomic coordinates of all multiple-mutated proteins, which are candidates for solutions, so as to obtain an optimum solution, since the number of such candidates is enormous. For example, if a certain 10 residues in the amino acid sequence of a protein to be mutated are each replaced with any of 20 naturally occurring amino acids, the total number of combinations of these amino acids over the sequence is as enormous as 20 to the power of 10 ($20^{10}$). Therefore, it is difficult to calculate the atomic coordinates and protein characteristics of multiple-mutated proteins in a practical calculation time.

If only a portion of the above-described enormous number of candidates for solutions are taken into consideration, it is possible to calculate the atomic coordinates and protein characteristics of the multiple-mutated proteins in a practical calculation time. However, even if an optimum solution is selected from only a randomly extracted portion of candidates for solutions, there is no guarantee that it is the optimum solution out of all possible candidates for solutions.

An objective of the present invention is to provide a method for calculating an optimized solution of the amino acid sequences of multiple-mutated proteins without a reduction in calculation accuracy and in a practical calculation time, an apparatus for calculating an optimized solution of the amino acid sequences of multiple-mutated proteins, a program for executing calculation of an optimized solution of the amino acid sequences of multiple-mutated proteins, and a recording medium carrying a method for calculating an optimized solution of the amino acid sequences of multiple-mutated proteins, thereby solving the above-described problems. The present invention also relates to a computer program for executing calculation of an optimized solution of the amino acid sequences of multiple-mutated proteins and a transmission medium carrying the computer program. The present invention further relates to provision of a service utilizing a method for calculating an optimized solution of the amino acid sequences of multiple-mutated proteins.

## DISCLOSURE OF THE INVENTION

(Summary of the Invention)

In a method of the present invention, an optimization method using a genetic algorithm (hereinafter also referred to as GA) is applied to optimize the amino acid sequence of a multiple-mutated protein, in which the atomic coordinates of the three-dimensional structures of multiple-mutated proteins, which are candidates for solutions obtained by the GA, are subjected to optimization using a dead end elimination (DEE) algorithm, thereby achieving the above-described objectives.

According to one aspect of the present invention, a method for calculating an optimized solution of the amino acid sequences of multiple-mutated proteins, comprises the steps of searching the three-dimensional structural coordinates of amino acid side chains of the amino acid sequences of members of a multiple-mutated protein population based on the three-dimensional structure data of a template protein population using a dead end elimination algorithm, and executing structural energy minimization calculations for the members, thereby calculating the three-dimensional structural coordinates of an optimum multiple-mutated protein, calculating a characteristic value from the three-dimensional structural coordinates of the optimum multiple-mutated protein, and applying a genetic algorithm to the multiple-mutated protein population to calculate the members which optimize the characteristic value. In one embodiment, the step of calculating the three-dimensional structural coordinates of the optimum multiple-mutated protein is carried out under a constraint that the three-dimensional structure of the template protein is generally maintained.

According to a preferable aspect of the present invention, a method for calculating an optimized solution of the amino acid sequences of multiple-mutated proteins, comprises the steps of (a) inputting sequence data and three-dimensional structure data of a template protein population, (b) calculating a characteristic value of each member in the template protein population based on the sequence data and the three-dimensional structure data of the template protein population, (c) inputting calculation parameters and a desired characteristic value to be used in the algorithm, (d) applying a genetic algorithm to the template protein population to generate a multiple-mutated protein population based on the calculation parameters, the desired characteristic value and the three-dimensional structure data and the characteristic value of each member in the template protein population, (e) applying a dead end elimination algorithm to amino acid side chains of amino acid residues of each member in the multiple-mutated protein population to optimize the conformations of the amino acid side chains, and carrying out energy minimization calculations, (f) calculating three-dimensional structure data and characteristic value of each member having a minimized energy in the multiple-mutated protein population. (g) determining whether or not steps (h) to (j) are to be carried out based on the calculation parameters, the desired characteristic value, the three-dimensional structure data and the characteristic value of each member in the template protein population, and the three-dimensional structure data and the characteristic value of each member in the multiple-mutated protein population, (h) when in step (g) it is determined that steps (h) to (j) are carried out, applying a genetic algorithm to the template protein population to generate a new multiple-mutated protein population based on the calculation parameters, the desired characteristic value and the characteristic value of the template protein population, and the characteristic value of each member in the multiple-mutated protein populations which have been generated, (i) applying the dead end elimination algorithm to amino acid side chains of amino acid residues of each member in the new multiple-mutated protein population to optimize the conformations of the amino acid side chains, and carrying out energy minimization calculations, (j) calculating three-dimensional structure data and a characteristic value of each member having a minimized energy in the new multiple-mutated protein population, (k) determining whether or not steps (h) to (j) are carried out based on the calculation parameters, the desired characteristic value, the characteristic value of the template protein population, and the characteristic value of each member in all of the multiple-mutated protein populations which have been generated, (1) selecting a member having the desired characteristic value from the characteristic values of the members in the template protein population and the characteristic values of the members in all of the multiple-mutated protein populations which have been generated, and (m) outputting the sequence data and the characteristic value of the selected member. In one embodiment, the sequence data of the template protein population is of amino acid sequence and/or nucleic acid sequence. In another embodiment, the three-dimensional structure data of the template protein population includes at least one selected from the group consisting of atomic coordinate data, molecular topology data, and molecular force field constants. In still another embodiment, the template protein population includes one member. In another embodiment, the template protein population includes at least two members. In another embodiment, the characteristic value or the desired characteristic value includes at least one data selected from the group consisting of empirical molecular mechanics potential, semi-empirical quantum mechanics potential, non-empirical quantum mechanics potential, electromagnetic potential, and solvation potential and structural entropy. In another embodiment, the calculation parameters are calculation parameters for the genetic algorithm. In another embodiment, the calculation parameters include a characteristic value which is a criterion for the determination in step (g). In another embodiment, the calculation parameters include information for specifying the conformations of amino acids to be mutated. In another embodiment, the dead end elimination algorithm is applied to at least one of the amino acid residues. In another embodiment, the dead end elimination algorithm is applied to all of the amino acid residues. In another embodiment, a protein characteristic to be modified is selected from thermal stability, chemical stability, chemical selectivity to a substrate, stereoselectivity to a substrate, and optimal pH value. In another embodiment, the amino acid sequence is selected from the group consisting of naturally occurring amino acids, chemically modified amino acids, and non-naturally occurring amino acids. In another embodiment, each member of the multiple-mutated protein population is a molecular complex including at least one protein comprising a plurality of homologous molecules, a plurality of heterologous molecules, or a combination thereof.

According to another aspect of the present invention, an apparatus for calculating an optimized solution of the amino acid sequences of multiple-mutatedproteins, comprises means for searching the three-dimensional structural coordinates of amino acid side chains of the amino acid sequences of members of amultiple-mutated protein population based on the three-dimensional structure data of a template protein population using a dead end elimination algorithm, and executing structural energy minimization calculations for the members, thereby calculating the three-dimensional structural coordinates of an optimum multiple-mutated protein, means for calculating a characteristic value from the three-dimensional structural coordinates of the optimum multiple-mutated protein, and means for applying a genetic algorithm to the multiple-mutated protein population to calculate the members which optimize the characteristic value. In one embodiment, the means for calculating the three-dimensional structural coordinates of the optimum multiple-mutated protein is carried out under a constraint that the three-dimensional structure of the template protein is generally maintained.

According to another preferable aspect of the present invention, an apparatus for calculating an optimized solution of the amino acid sequences of multiple-mutated proteins, comprises:

(1) an input section;
(2) a calculation section; and
(3) an output section,

wherein the input section comprises:

(a) means for inputting sequence data and three-dimensional structure data of a template protein population; and
(c) means for inputting calculation parameters and a desired characteristic value to be used in the algorithm;

the calculation section comprises:

(c) means for calculating a characteristic value of each member in the template protein population based on the sequence data and the three-dimensional structure data of the template protein population,
(d) means for applying a genetic algorithm to the template protein population to generate a multiple-mutated protein population based on the calculation parameters, the desired characteristic value and the three-dimensional structure data and the characteristic value of each member in the template protein population;
(e) means for applying a dead end elimination algorithm to amino acid side chains of amino acid residues of each member in the multiple-mutated protein population to optimize the conformations of the amino acid side chains, and carrying out energy minimization calculations;
(f) means for calculating three-dimensional structure data and characteristic value of each member having a minimized energy in the multiple-mutated protein population, and storing the calculated three-dimensional structure data and characteristic value;
(g) means for determining whether or not the steps for generating a population carried out by the means (d) to (f) are to be carried out based on the calculation parameters, the desired characteristic value, the three-dimensional structure data and the characteristic value of each member in the template protein population, and the three-dimensional structure data and the characteristic value of each member in the multiple-mutated protein population; and
(i) selecting a member having the desired characteristic value from the characteristic values of the members in the template protein population and the characteristic values of the members of the multiple-mutated protein populations,

wherein the output section comprises means for outputting the sequence data and characteristic value of the selected member.

In one embodiment, the sequence data of the template protein population is of amino acid sequence and/or nucleic acid sequence. In another embodiment, the three-dimensional structure data of the template protein population includes at least one selected from the group consisting of atomic coordinate data, molecular topology data, and molecular force field constants. In another embodiment, the template protein population includes one member. In another embodiment, the template protein population includes at least two members. In another embodiment, the characteristic value or the desired characteristic value includes at least one data selected from the group consisting of empirical molecular mechanics potential, semi-empirical quantum mechanics potential, non-empirical quantum mechanics potential, electromagnetic potential, and solvation potential and structural entropy. In another embodiment, the calculation parameters are calculation parameters for the genetic algorithm. In another embodiment, the calculation parameters include a characteristic value which is a criterion for the determination in step (g). In another embodiment, the calculation parameters include information for specifying the conformations of amino acids to be mutated. In another embodiment, the dead end elimination algorithm is applied to at least one of the amino acid residues. In another embodiment, the dead end elimination algorithm is applied to all of the amino acid residues. In another embodiment, a protein characteristic to be modified is selected from thermal stability, chemical stability, chemical selectivity to a substrate, stereoselectivity to a substrate, and optimal pH value. In another embodiment, the amino acid sequence is selected from the group consisting of naturally occurring amino acids, chemically modified amino acids, and non-naturally occurring amino acids. In another embodiment, each member of the multiple-mutated protein population is a molecular complex including at least one protein comprising a plurality of homologous molecules, a plurality of heterologous molecules, or a combination thereof. In another embodiment, the apparatus further comprises a data storage section.

According to another aspect of the present invention, a computer readable recording medium recording a program is provided for executing a method for calculating an optimized solution of the amino acid sequences of multiple-mutated proteins based on input data. The method comprises the steps of searching the three-dimensional structural coordinates of amino acid side chains of the amino acid sequences of members of a multiple-mutated protein population based on the three-dimensional structure data of a template protein population using a dead end elimination algorithm, and executing structural energy minimization calculations for the members, thereby calculating the three-dimensional structural coordinates of an optimum multiple-mutated protein, calculating a characteristic value from the three-dimensional structural coordinates of the optimum multiple-mutated protein, and applying a genetic algorithm to the multiple-mutated protein population to calculate the members which optimize the characteristic value.

According to another aspect of the present invention, a computer readable recording medium recording a program is provided for executing a method for calculating an optimized solution of the amino acid sequences of multiple-mutated proteins based on input data. The method comprises the steps of

(a) inputting sequence data and three-dimensional structure data of a template protein population;

(b) calculating a characteristic value of each member in the template protein population based on the sequence data and the three-dimensional structure data of the template protein population;

(c) inputting calculation parameters and a desired characteristic value to be used in the algorithm;

(d) applying a genetic algorithm to the template protein population to generate a multiple-mutated protein population based on the calculation parameters, the desired characteristic value and the three-dimensional structure data and the characteristic value of each member in the template protein population;

(e) applying a dead end elimination algorithm to amino acid side chains of amino acid residues of each member in the multiple-mutated protein population to optimize the conformations of the amino acid side chains, and carrying out energy minimization calculations;

(f) calculating three-dimensional structure data and characteristic value of each member having a minimized energy in the multiple-mutated protein population;

(g) determining whether or not steps (h) to (j) are to be carried out based on the calculation parameters, the desired characteristic value, the three-dimensional structure data and the characteristic value of each member in the template protein population, and the three-dimensional structure data and the characteristic value of each member in the multiple-mutated protein population;

(h) when in step (g) it is determined that steps (h) to (j) are carried out, applying a genetic algorithm to the template protein population to generate a new multiple-mutated protein population based on the calculation parameters, the desired characteristic value and the characteristic value of the template protein population, and the characteristic value of each member in the multiple-mutated protein populations which have been generated;

(i) applying the dead end elimination algorithm to amino acid side chains of amino acid residues of each member in the new multiple-mutated protein population to optimize the conformations of the amino acid side chains, and carrying out energy minimization calculations;

(j) calculating three-dimensional structure data and a characteristic value of each member having a minimized energy in the new multiple-mutated protein population;

(k) determining whether or not steps (h) to (j) are carried out based on the calculation parameters, the desired characteristic value, the characteristic value of the template protein population, and the characteristic value of each member in all of the multiple-mutated protein populations which have been generated;

(l) selecting a member having the desired characteristic value from the characteristic values of the members in the template protein population and the characteristic values of the members in all of the multiple-mutated protein populations which have been generated; and
(m) outputting the sequence data and the characteristic value of the selected member.

According to another aspect of the present invention, a transmission medium is provided for transmitting a program for executing a method for calculating an optimized solution of the amino acid sequences of multiple-mutated proteins based on input data. The method comprises the steps of:

searching the three-dimensional structural coordinates of amino acid side chains of the amino acid sequences of members of a multiple-mutated protein population based on the three-dimensional structure data of a template protein population using a dead end elimination algorithm, and executing structural energy minimization calculations for the members, thereby calculating the three-dimensional structural coordinates of an optimum multiple-mutated protein;
calculating a characteristic value from the three-dimensional structural coordinates of the optimum multiple-mutated protein; and
applying a genetic algorithm to the multiple-mutated protein population to calculate the members which optimize the characteristic value.

According to another preferable aspect of the present invention, a transmission medium is provided for transmitting a program for executing a method for calculating an optimized solution of the amino acid sequences of multiple-mutated proteins based on input data. The method comprises the steps of:

(a) inputting sequence data and three-dimensional structure data of a template protein population;
(b) calculating a characteristic value of each member in the template protein population based on the sequence data and the three-dimensional structure data of the template protein population;
(c) inputting calculation parameters and a desired characteristic value to be used in the algorithm;
(d) applying a genetic algorithm to the template protein population to generate a multiple-mutated protein population based on the calculation parameters, the desired characteristic value and the three-dimensional structure data and the characteristic value of each member in the template protein population;
(e) applying a dead end elimination algorithm to amino acid side chains of amino acid residues of each member in the multiple-mutated protein population to optimize the conformations of the amino acid side chains, and carrying out energy minimization calculations;
(f) calculating three-dimensional structure data and characteristic value of each member having a minimized energy in the multiple-mutated protein population;
(g) determining whether or not steps (h) to (j) are to be carried out based on the calculation parameters, the desired characteristic value, the three-dimensional structure data and the characteristic value of each member in the template protein population, and the three-dimensional structure data and the characteristic value of each member in the multiple-mutated protein population;
(h) when in step (g) it is determined that steps (h) to (j) are carried out, applying a genetic algorithm to the template protein population to generate a new multiple-mutated protein population based on the calculation parameters, the desired characteristic value and the characteristic value of the template protein population, and the characteristic value of each member in the multiple-mutated protein populations which have been generated;
(i) applying the dead end elimination algorithm to amino acid side chains of amino acid residues of each member in the new multiple-mutated protein population to optimize the conformations of the amino acid side chains, and carrying out energy minimization calculations;
(j) calculating three-dimensional structure data and a characteristic value of each member having a minimized energy in the new multiple-mutated protein population;
(k) determining whether or not steps (h) to (j) are carried out based on the calculation parameters, the desired characteristic value, the characteristic value of the template protein population, and the characteristic value of each member in all of the multiple-mutated protein populations which have been generated;
(l) selecting a member having the desired characteristic value from the characteristic values of the members in the template protein population and the characteristic values of the members in all of the multiple-mutated protein populations which have been generated; and
(m) outputting the sequence data and the characteristic value of the selected member.

According to another aspect of the present invention, a program is provided for executing a method for calculating an optimized solution of the amino acid sequences of multiple-mutated proteins based on input data. The program causesthe computer to execute the processes of:

searching the three-dimensional structural coordinates of amino acid side chains of the amino acid sequences of members of a multiple-mutated protein population based on the three-dimensional structure data of a template protein population using a dead end elimination algorithm, and executing structural energy minimization calculations for the members, thereby calculating the three-dimensional structural coordinates of an optimum multiple-mutated protein;

calculating a characteristic value from the three-dimensional structural coordinates of the optimum multiple-mutated protein; and

applying a genetic algorithm to the multiple-mutated protein population to calculate the members which optimize the characteristic value.

According to another preferable aspect of the present invention, a program is provided for executing a method for calculating an optimized solution of the amino acid sequences of multiple-mutated proteins based on input data. The program causes the computer to execute the processes of:

(a) inputting sequence data and three-dimensional structure data of a template protein population, and thereafter, calculating a characteristic value of eachmember in the template protein population based on the sequence data and the three-dimensional structure data of the template protein population;

(b) inputting calculation parameters and a desired characteristic value to be used in the algorithm, and thereafter, applying a genetic algorithm to the template protein population to generate a multiple-mutated protein population based on the calculation parameters, the desired characteristic value and the three-dimensional structure data and the characteristic value of each member in the template protein population;

(c) applying a dead end elimination algorithm to amino acid side chains of amino acid residues of each member in the multiple-mutated protein population to optimize the conformations of the amino acid side chains, and carrying out energy minimization calculations;

(d) calculating three-dimensional structure data and characteristic value of each member having a minimized energy in the multiple-mutated protein population;

(e) determining whether or not steps (h) to (j) are to be carried out based on the calculation parameters, the desired characteristic value, the three-dimensional structure data and the characteristic value of each member in the template protein population, and the three-dimensional structure data and the characteristic value of each member in the multiple-mutated protein population;

(f) when in step (e) it is determined that steps (h) to (j) are carried out, applying a genetic algorithm to the template protein population to generate a new multiple-mutated protein population based on the calculation parameters, the desired characteristic value and the characteristic value of the template protein population, and the characteristic value of each member in the multiple-mutated protein populations which have been generated;

(g) applying the dead end elimination algorithm to amino acid side chains of amino acid residues of each member in the new multiple-mutated protein population to optimize the conformations of the amino acid side chains, and carrying out energy minimization calculations;

(h) calculating three-dimensional structure data and a characteristic value of each member having a minimized energy in the new multiple-mutated protein population;

(i) determining whether or not steps (f) to (h) are carried out based on the calculation parameters, the desired characteristic value, the characteristic value of the template protein population, and the characteristic value of each member in all of the multiple-mutated protein populations which have been generated;

(j) selecting a member having the desired characteristic value from the characteristic values of the members in the template protein population and the characteristic values of the members in all of the multiple-mutated protein populations which have been generated; and

(k) outputting the sequence data and the characteristic value of the selected member.

The present invention further relates to a method for providing a service for calculating an optimized solution of the amino acid sequences of multiple-mutated proteins based on input data over a network. The method comprises:

the step of inputting three-dimensional structure data, amino acid sequence data and calculation parameters of a template protein population to a server, and

the step of the server searching the three-dimensional structural coordinates of amino acid side chains of the amino acid sequences of members of a multiple-mutated protein population based on the three-dimensional structure data of a template protein population using a dead end elimination algorithm, and executing structural energy minimization calculations for the members, thereby calculating the three-dimensional structural coordinates of an optimum multiple-mutated protein;

the step of the server calculating a characteristic value from the three-dimensional structural coordinates of the optimum multiple-mutated protein; and
the step of the server applying a genetic algorithm to the multiple-mutated protein population to calculate the members which optimize the characteristic value.

According to another preferable aspect of the present invention, a method is provided for providing a service for calculating an optimized solution of the amino acid sequences of multiple-mutated proteins based on input data over a network. The method comprises:

(a) the step of inputting sequence data and three-dimensional structure data of a template protein population;
(b) the step of a server calculating a characteristic value of each member in the template protein population based on the sequence data and the three-dimensional structure data of the template protein population;
(c) the step of inputting calculation parameters and a desired characteristic value to be used in the algorithm;
(d) the step of the server applying a genetic algorithm to the template protein population to generate a multiple-mutated protein population based on the calculation parameters, the desired characteristic value and the three-dimensional structure data and the characteristic value of each member in the template protein population;
(e) the step of the server applying a dead end elimination algorithm to amino acid side chains of amino acid residues of each member in the multiple-mutated protein population to optimize the conformations of the amino acid side chains, and carrying out energy minimization calculations;
(f) the step of the server calculating three-dimensional structure data and characteristic value of each member having a minimized energy in the multiple-mutated protein population;
(g) the step of the server determining whether or not steps (h) to (j) are to be carried out based on the calculation parameters, the desired characteristic value, the three-dimensional structure data and the characteristic value of each member in the template protein population, and the three-dimensional structure data and the characteristic value of each member in the multiple-mutated protein population;
(h) the step of the server, when in step (g) it is determined that steps (h) to (j) are carried out, applying a genetic algorithm to the template protein population to generate a new multiple-mutated protein population based on the calculation parameters, the desired characteristic value and the characteristic value of the template protein population, and the characteristic value of each member in the multiple-mutated protein populations which have been generated;
(i) the step of the server applying the dead end elimination algorithm to amino acid side chains of amino acid residues of each member in the new multiple-mutated protein population to optimize the conformations of the amino acid side chains, and carrying out energy minimization calculations;
(j) the step of the server calculating three-dimensional structure data and a characteristic value of each member having a minimized energy in the new multiple-mutated protein population;
(k) the step of the server determining whether or not steps (h) to (j) are carried out based on the calculation parameters, the desired characteristic value, the characteristic value of the template protein population, and the characteristic value of each member in all of the multiple-mutated protein populations which have been generated;
(l) the step of the server selecting a member having the desired characteristic value from the characteristic values of the members in the template protein population and the characteristic values of the members in all of the multiple-mutated protein populations which have been generated; and
(m) the step of the server outputting the sequence data and the characteristic value of the selected member.

BRIEF DESCRIPTION OF THE DRAWINGS

Figure **1** is a flowchart of a mutated protein design method using a genetic algorithm.

Figure **2** shows a detailed exemplary configuration of a mutated protein sequence control section.

Figure **3** shows a detailed exemplary configuration of a mutated protein three-dimensional structure optimization apparatus and a mutated protein characteristic value calculation section.

Figure **4** shows an exemplary implemented configuration of the present invention.

Figure **5A** is a diagram for explaining the results of an example.

Figure 5B is the continuation of the diagram of Figure **5A.**

Figure **6** shows an exemplary configuration of a computer **500** for executing the present invention.

BEST MODE FOR CARRYING OUT THE INVENTION

Hereinafter, the present invention will be described in detail.

In the present invention, a genetic algorithm is applied to generate genetic mutations, and DEE is employed to optimize the coordinates of a generated mutant.

A "genetic algorithm (GA)" is an algorithm for optimization, in which adaptation to an environment, which is a major challenge in evolution, is viewed as processing of a genetic information, and which is a molecular process in the overall evolutionary theory. Specifically, genetic algorithm is an algorithm for adaptation, which is based on learning called self-organization resulting from the complexed combination of recognition of a target, interaction with the environment, and memory storing properties observed in organisms, and the basis of the information is heredity (Y. Yonezawa, "Identeki-Arugorizum - Shinkariron-no-Jhohokagaku" [Genetic Algorithm - Information Science of Evolutionary Theory], Morikita-Shuppan, 1993).

Organisms "recognize" a "target", which should be achieved in order to survive, based on an instinct for survival. To "recognize a target", organisms may utilize information useful for reference and criteria of selection (or deletion) in the evolutionary process. Organisms may "interact with their environment", and then "memorize and store" effective conditions in order to predict an environment effective for their survival. As a result, the organisms may perform the activities, "learning and adaptation". In learning and adaptation, a high-level phenomenon "self-organization", which is the greatest characteristic of organisms, is achieved.

(Specific description of a genetic algorithm)

Hereinafter, the fundamental scheme of a genetic algorithm will be described.

The genetic algorithm utilizes two processes, sexual reproduction and natural selection, which are used by organisms. In the sexual reproduction of organisms, homologous chromosomes pair as represented by fertilization of a sperm and an egg. Thereafter, crossover occurs any site in a chromosome, causing gene exchange, i.e., gene recombination. Gene recombination achieves diversification of information more effectively and efficiently than mutation. In natural selection, in which individuals diversified by sexual reproduction or the like are caused to remain and become next-generation surviving organisms, i.e., adaptive organisms, are determined. Unlike conventional algorithms, the genetic algorithm is characterized in that the risk of a solution falling into a local optimum is significantly reduced.

The basic scheme of the genetic algorithm will be described. The entire genetic algorithm is roughly divided into the following eight processes:

(1) Determination of genotype;
(2) Generation of population;
(3) Evaluation of individuals;
(4) Selection (deletion);
(5) Reproduction;
(6) crossover (recombination);
(7) Mutation; and
(8) Evaluation of groups.

A population generated in (2) is subjected to selection in (3) and (4), and diversified in (5) to (7). The resulting solutions are evaluated in (8). Depending on the results, (3) to (7) (herein referred to as one "generation") are repeated. The above-described generation of new individuals and change of generation are the basic scheme of the genetic algorithm. In this manner, in the genetic algorithm, a population of events to be solved (optimum solution region: a region having a plurality of solutions, but not a sole solution) are artificially evolved (i.e., optimum adaptation) so that the solution approaches a true optimum value for the entire population. Here, at least one of processes (4) to (7) may be omitted.

Next, each process in the genetic algorithm will be described.

(1) Determination of genotype

In this process, a genotype is determined. An event or system is modeled (i.e., division of the event into components, definition thereof, and definition between each component) and the model is represented by symbols. Therefore, the event can be described by DNAs and amino acids. Representatively, the event is represented by, but is not limited to, binary digits (bit), numerical values, characters, or the like. If the modeling of an event is not appropriate for the above-described symbolic representation, the event is not adapted to GA.

(2) Generation of population

Diversity is generated. In principle, a number of slightly different individuals are generated. A random method and a rule method may be used. In the random method, an initial value is based on random number generation. In the rule method, an initial value is based on a predetermined criterion.

In this process, individuals are ranked in terms of fitness to an environment from high to low. Examples of evaluation parameters for proteins include, but are not limited to, empirical molecular mechanics potential, semi-empirical quantum mechanics potential, non-empirical quantum mechanics potential, electromagnetic potential, solvation potential, structural entropy, pI (isoelectric point), and the like. These evaluation parameters may be directly or indirectly related to the biochemical properties of protein.

(4) Selection (deletion)

Selection is a process for selecting individuals which remain in the next generation based on the evaluation values resulting from an evaluation function in (3). Therefore, some individuals are deleted depending on the evaluation by the evaluation function. Selection is roughly divided into three categories, depending on the manner of deletion.

(a) Random method (roulette method): individuals are first rejected that have numerical values of fitness less than a predetermined value, and the remaining individuals are randomly screened.

(b) fitness ranking method (ranking method): individuals are not rejected depending on the numerical values of fitness. Instead, individual members are ranked in the terms of fitness and are each given selection probabilities depending on their rank. The individuals are selected based on their probabilities.

(c) High fitness choice method (elite conservation method): the individual which has the greatest fitness in a group to which the individual belongs is unconditionally selected.

(5) Reproduction

In this process, the reduced number of individuals in (4) are subjected to reproduction. Reproduction is conducted in a predetermined manner so that a predetermined proportion of individuals are extracted from the overall individuals after the selection and are then subjected to reproduction. This process leads to an increase in the average value of fitness in the entire population. Examples of the reproduction include causing individuals having high evaluation values to reproduce preferentially, causing individuals to reproduce in proportion to the proportion of remaining individuals.

(6) Crossover (recombination)

Crossover mimics a crossover event in gene recombination. In this process, particular symbols in one individual are replaced with corresponding symbols in another individual. When only selection is performed, no individual having an evaluation value exceeding the highest evaluation value in the population is newly generated. With this process, it is possible to generate an individual having a still higher evaluation value.

Crossover is roughly divided into one-point crossover, multi-point crossover, uniform crossover, order crossover, cycle crossover, and partially matched crossover.

(7) Mutation

Mutation is a process in which particular sites of individuals are changed with a predetermined probability. Species to be changed may be all naturally occurring amino acids (20 types), or a group of particular amino acids. Alternatively, non-naturally occurring amino acids or modified amino acids may be changed. In selection or crossover, the resultant highest value is constrained by the initial values. With mutation, individuals having high fitness values can be generated without depending on the initial values. Mutation is divided into translocation, overlapping, inversion, insertion, deletion, and the like.

(8) Evaluation of organism population

In this process, the individual population obtained by the above-described processes is evaluated using predetermined characteristic parameters. In this case, a termination condition, i.e., whether or not the above-described processes are to be repeated is judged.

The above-described processes are repeated over a certain number of generations, thereby achieving the genetic algorithm.

(Dead end elimination (DEE))

Dead end elimination is a method for predicting the optimum value, or global minimum energy conformation (GMEC) of the side chain structure of amino acids of a protein (Desmet, J. et al. (1992), 356, 539-542; and Desmet, J. et al. (1994), The Protein Folding Problem and Tertiary Structure Prediction, Merz et al. Ed., Birkhaeuser Boston, 307-337). If a side chain can be approximated by rotamers, the structure of the side chain as it is present at an assumed site in the principal chain structure can be predicted by a combination of rotamers. For example, assuming that a protein consists of 100 amino acid residues and there are three isomers for each residue, there are $3^{100}$ combinations. In this case, if one isomer for a certain residue causes the overall energy to be very disadvantageous irrespective of the other residues in any isometric side chain structure, any structure including the particular residue may be omitted from all subjects to be evaluated. This judgement is strictly conducted in accordance with dead end theorem. With this method, for most proteins, if a principal chain structure is provided, its optimum side chain structure can be determined.

Specifically, the dead end theorem will be described.

Statistical analysis of the structure of protein crystal has revealed that the twist of the carbon chain in an amino acid side chain of protein has mainly three types of conformation: gauche (+) (+60°), gauche (-) (-60°), and trans (180°). Of the various amino acids, tryptophan or tyrosine having a benzene ring have a $\chi^2$ angle which is close to 90°, histidine and tryptophan are +90° or -90° in conformation. Generally, the longer the side chain of amino acids, the larger the number of possible types of conformation, i.e., rotamer. For example, lysine has about 51 types of conformations and arginine has about 55 types of conformation (Desmet et al. (1992) supra).

The potential energy functions or evaluation functions of various assumed rotamers are generated. These functions include, representatively, terms related to the strength of a bond, terms related to a bond angle, periodic functions related to the twist of a bond, the Lennard-Jones potential of a nonbonded atom pair, the potential of a hydrogen bond, and the Coulomb function of electrons. The energy of a rotamer is calculated using such an evaluation function and is employed as described below.

The objective of the dead end elimination algorithm is to calculate the GMEC of a predetermined set of rotatable side chains. In this algorithm, a fixed reference structure referred to as a template is compared with structures containing various rotamers. Such a template includes (1) the atoms of a principal chain, (2) Cβ atoms, (3) possible ligands (e.g., water molecules, metal ions, substrates, heme groups, and the like), (4) interactive proteins (e.g., other subunits in the case of a multimer), and (5) side chains unnecessary for modeling.

When, for a particular rotamer $i_r$ in a side chain of a certain residue i, there is another rotamer $i_t$, whether or not the rotamer $i_r$ is omitted from consideration is determined based on the following calculation. For a rotamer in a side chain of a certain residue, the unique self-energy of the side chain is calculated using an evaluation function. Further, the interaction energy of a fixed atom of a template and an atom in the side chain is calculated. The sum of these two energies is referred to as an "intrinsic energy" ($E(i_r)$ where $i_r$ is a particular rotamer in a certain residue) of the rotamer. Thereafter, the interaction energy of an atom in a rotamer and an atom in another rotamer is integrated over all residues, and the resultant value is referred to as the "non-bonded pair interaction energy" ($\Sigma_j E(i_r j_s)$ where $j_s$ is a particular rotamer of a residue different from i). The minimum integral of the non-bonded pair interaction energy of each residue is referred to as the "minimum non-bonded pair interaction energy" ($\Sigma_j min_s E(i_r j_s)$). The maximum integral of the non-bonded pair interaction energy of each residue is referred to as the "maximum non-bonded pair interaction energy" ($\Sigma_j max_s E(i_r j_s)$).

Here, the following expression is established:

$$E(i_r) + \Sigma_j min_s E(i_r j_s) > E(i_t) + \Sigma_j max_s E(i_r j_s)$$

wherein $i \neq j$.

Specifically, when the sum of the intrinsic energy and minimum non-bonded pair interaction energies of a certain rotamer $i_r$ is greater than the sum of the intrinsic energy and maximum non-bonded pair interaction energies of another rotamer $i_t$, the rotamer $i_r$ is not part of GMEC. Therefore, the rotamer $i_r$ is omitted from calculation of GMEC. By calculating each rotamer successively, all rotamers that are not suitable for GMEC are omitted, and then GMEC is calculated.

Since unnecessary candidates can be efficiently omitted from calculation, the DEE algorithm can be used to calculate GMEC with a significantly reduced calculation amount.

(Energy minimization calculation)

Next, an energy minimization calculation will be described.

Energy minimization is a method for calculating the stable structure of a system, such as protein structure. In energy minimization, a stable local structure is obtained, which is not far from the starting structure.

In energy minimization, initial coordinates are first given. Thereafter, the initial coordinates are slightly changed in a direction such that energy is expected to be decreased so as to obtain a next set of initial coordinates. This step is repeated. When a change in structure, a change in energy, and force become sufficiently small, the repetition is stopped, so that a structure having a minimum energy is obtained (see Gendai Kagaku, special issue 13, "Shinyaku-no-ridogyenereshon [Lead Generation of New Drugs]", Chapter 13, molecular dynamics design system, Tokyo Kagaku Dojin).

The method is represented using vectors by:

$$\tilde{r}_{n+1} = \tilde{r}_n + \delta_n$$

where r with - indicates coordinates in step n or n+1, and $\delta$ indicates a change in the coordinates.

To obtain $\delta$, representatively, a steepest descent method, a conjugate gradient method, a Newton-Raphson method (NR method), or an adaptive Newton-Raphson method (ABNR method) may be used.

In a steepest descent method, $\delta$ is obtained based on the gradient of potential energy.

$$\tilde{\delta} = -k_n (\nabla_n E)$$

While energy is efficiently decreased at a place far away from a minimum point, convergence tends to become slower near a minimum point. $k_n$ is a parameter used for search on lines.

In the conjugate gradient method, coordinates in the next step are calculated based on an energy gradient and the energy gradient in the previous step. In general. better convergence is believed to be obtained than that in the steepest descent method.

$$\tilde{\delta}_n = -\tilde{g}_n + \delta_{n-1} \frac{|g_n|^2}{|g_{n-1}|^2}$$

$$\tilde{r}_{n+1} = \tilde{r}_n + \tilde{\alpha} \delta_n$$

where $\delta = -k_n(\nabla_n E)$, and $\alpha$ is a parameter whose optimum value is determined by a simple search on lines.

In the NR method, $\delta$ is calculated based on a first-order differential (gradient) and further on a second-order differential matrix (curvature).

$$\tilde{\delta}_n = -\tilde{H}_n^{-1}\tilde{g}_n$$

$$[\tilde{H}_n]_{k,1} = \left(\frac{\partial^2 E}{\partial r_k \partial r_1}\right) r_n$$

In this method, although convergence is fast in the vicinity of a minimum point, it disadvantageously takes a long time to calculate a matrix of force constants and the inverse matrix thereof. Further, a large storage capacity is required. Therefore, it is difficult to apply this method to macromolecules.

Whereas in the NR method base vectors are solved in a complete space, the ABNR method is a simple method which solves base vectors in a sub-space, and can be applied to macromolecules. In order to find a portion having the greatest movement in the previous steps, base vectors in n-th step are generated from position vectors in the last p+1 steps.

$$\tilde{r}_n = \tilde{r}_{n-1} + \tilde{r}_{n-p-1}$$
$$\tilde{r}_n{}^1 = \tilde{r}_{n-1} + \tilde{r}_{n-p}$$
$$\vdots$$
$$\tilde{r}_n = \tilde{r}_{n-1} + \tilde{r}_{n-2}$$

   p is usually a value of 4 to 10. The second-order differential matrix is generated from reduced base vectors and first-order vectors, so that the size of matrix is significantly reduced and therefore calculation time and storage capacity may be small. The ABNR method has advantages of taking the calculation speed of the first-order differential method and the features, that important vectors are taken, of the second-order differential method in the NR method. The first p+1 steps are calculated by a steepest descent method, and then the ABNR method is used.

(Definitions)

Hereinafter, a part of the major terms used herein will be defined.

The term "template protein population" refers to a population of proteins which are the basis of calculation when it is herein used in genetic algorithms. A template protein population includes, but is not limited to, at least one protein, typically at least two proteins (i.e., members), preferably at least four proteins, more preferably proteins which belong to the same identified protein superfamily. The term "multiple-mutated protein population" refers to a population of proteins into which multiple mutations have been introduced. A multiple-mutated protein population may include a plurality of homologous molecules, a plurality of heterologous molecules, or a combination thereof. Preferably, a multiple-mutated protein population consists of a plurality of homologous molecules. Further, preferably, a multiple-mutated protein population consists of a plurality of heterologous molecules. Furthermore, preferably, a multiple-mutated protein population consists of a combination of a plurality of homologous molecules and a plurality of heterologous molecules. Each member of the multiple-mutated protein population may be a molecular complex including at least one protein including a plurality of homologous molecules, a plurality of heterologous molecules, or a combination thereof. The term "mutation" refers to a change in the amino acid sequence of a protein, i.e., amino acid substitution, deletion, insertion, or modification in the amino acid sequence of a protein. The term "multiple-mutated" as used herein usually refers to multiple mutations, but may be a single mutation. The term "member" in a template protein population or a multiple-mutated protein population refers to a protein member which belongs to a corresponding population.

The term "sequence data" of a protein refers to the amino acid sequence data of the protein or the nucleic acid sequence data encoding the amino acid sequence. A nucleic acid sequence may be a known sequence or a putative sequence estimated from an amino acid sequence.

The term "three-dimensional structure data" of a protein refers to data relating to the three-dimensional structure of the protein. Examples of the three-dimensional structure data of a protein representatively include atomic coordinate data, molecular topology, and molecular force field constants. Atomic coordinate data is representatively obtained from X-ray crystallography or NMR structural analysis. Such atomic coordinate data may be obtained by newly conducting X-ray crystallography or NMR structural analysis, or is available from known database (e.g., protein · data · bank (PDB)). Atomic coordinate data may also be produced by modeling or calculation. The term "three-dimensional structure type" or "fold" as used herein refers to the arrangement of the secondary structure inside a protein in three-dimensional space or topology. A method of the present invention is carried out preferably under the constraint that the three-dimensional structure type of a template protein is approximately conserved.

Molecular topology may be calculated using a tool program which is commercially available or is freeware, or may be created by the user. Alternatively, a molecular topology calculation program attached to a commercially available molecular force field calculation program (e.g., prepar program attached to PRESTO, Protein Engineering Research Institute (PERI)) may be employed.

Molecular force field constants (or molecular force field potential) may be calculated with a commercially available or freeware tool program, or a program created by the user. Alternatively, molecular force field constant data attached to a commercially available molecular force field calculation program (e.g., AMBER, Oxford Molecular) may be employed.

The term "characteristic value" of a protein refers to a physicochemical property of the protein. A characteristic value may be calculated from sequence data and/or three-dimensional structure data. Examples of a characteristic value of a protein representatively include, but are not limited to, empirical molecular mechanics potential, semi-empirical quantum mechanics potential, non-empirical quantum mechanics potential, electromagnetic potential, solvation potential, and structural entropy. A characteristic value of a protein may be related to a biochemical characteristic of a protein. A characteristic value of a protein may be related directly or indirectly to biochemical characteristics, such as the thermal stability and chemical stability of a protein or polypeptide, such as enzymes or signal transduction proteins, the chemical selectivity to a substrate or stereoselectivity to a substrate of an enzyme, optimal pH, and the like. These direct or indirect relations may be easily recognized by those skilled in the art. Therefore, those skilled in the art may predetermine "desired characteristic values" and calculation parameters, depending on their purposes. The term "desired characteristic value" as used herein refers to a target value when a characteristic value of a protein is altered.

The term "calculation parameter" as used herein refers to a parameter required for executing a method of the present invention. A calculation parameter is representatively a parameter for a genetic algorithm. Such a calculation parameter includes a parameter involved in changing any one of the number of populations, the number of individuals in a population, the number of generations, a selection rate, a reproduction rate, a crossover rate, or a mutation rate, or a combination thereof. The term "the number of generations" as used herein refers to the number of repetitions of a genetic algorithm. A calculation parameter also includes a characteristic value which is a criterion for determining the repetition of a genetic algorithm. A calculation parameter also includes information used to determine the position of an amino acid to be mutated. Further, a calculation parameter includes a calculation parameter relating to the number of generations N, where N is the number of times at which the optimum value of a characteristic value of a protein which has been calculated N-1 times first becomes equal to that resulting from the $N^{th}$ calculation. Thus, a calculation parameter may be related directly or indirectly to a biochemical characteristic of a protein to be mutated. Therefore, by handling these calculation parameters appropriately, a protein having a desired biochemical characteristic, or a characteristic approximate to the desired biochemical characteristic may be produced.

According to one aspect of the present invention, a method for calculating an optimized solution of multiple-mutated proteins is provided.

A method of the present invention for calculating an optimized solution of the amino acid sequences of multiple-mutated proteins, comprises the steps of: searching the three-dimensional structural coordinates of amino acid side chains of the amino acid sequence of members of a multiple-mutated protein population based on the three-dimensional structure data of a template protein population using a dead end elimination algorithm, and executing structural energy minimization calculation for the members, thereby calculating the three-dimensional structural coordinates of an optimum multiple-mutated protein; calculating a characteristic value from the three-dimensional structural coordinates of the optimum multiple-mutated protein; and applying a genetic algorithm to the multiple-mutated protein population to optimize the characteristic value.

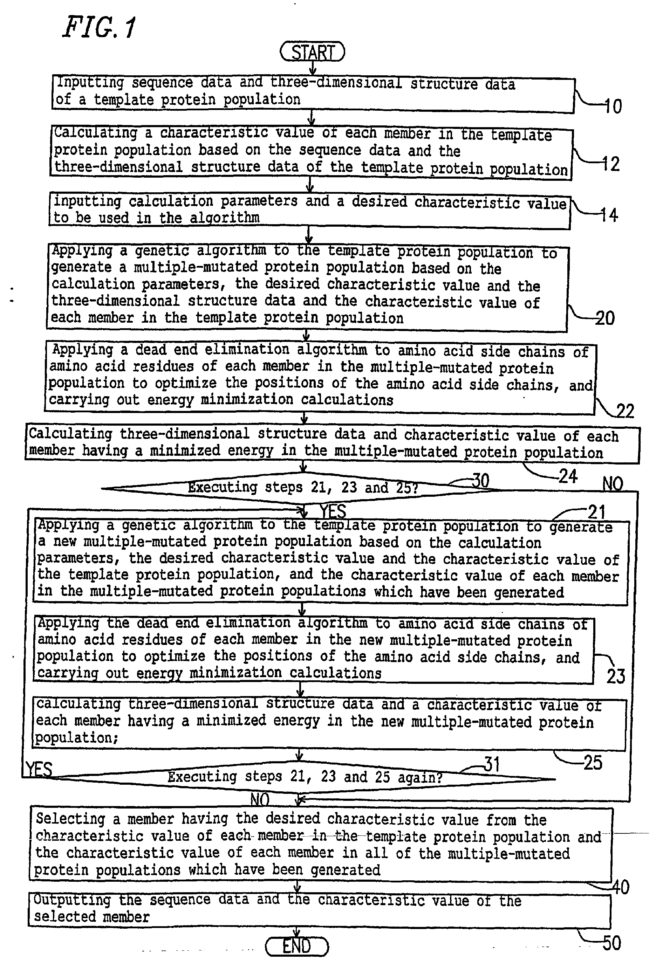

Figure 1 is an illustrative flowchart showing a method for calculating an optimized solution of multiple-mutated proteins. The method shown in Figure 1 is carried out with a computer 500.

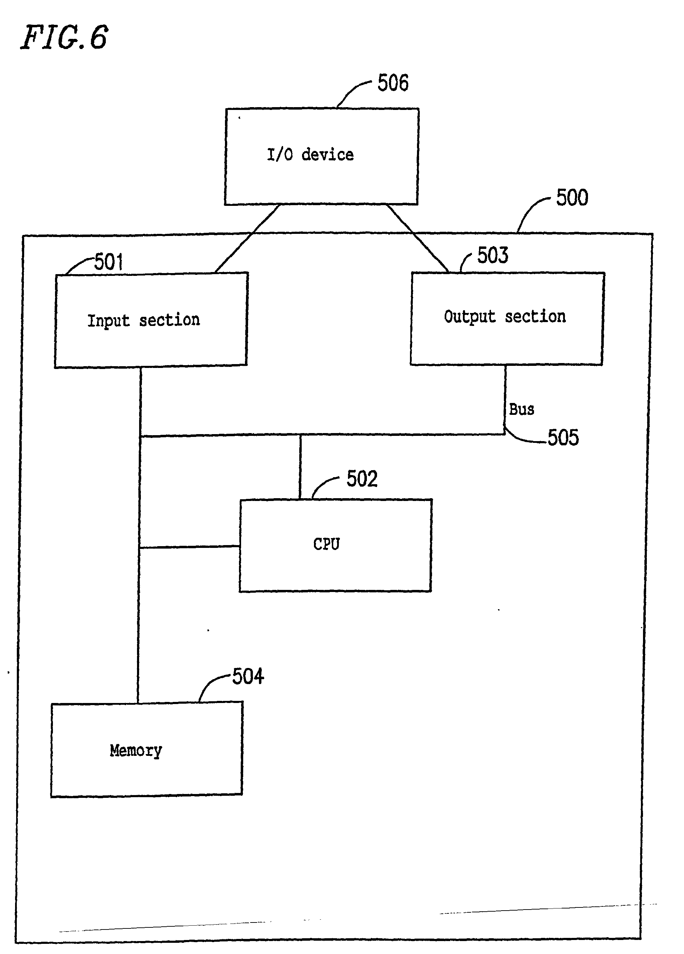

Figure 6 shows an exemplary configuration of the computer 500 which executes the method of the present invention for calculating an optimized solution of multiple-mutated proteins.

The computer 500 comprises an input section 501, a CPU 502, an output section 503, a memory 504, and a bus 505. The input section 501, the CPU 502, the output section 503, and the memory 504 are connected through the bus 505 to each other. The input section 501 and the output section 503 are connected to an I/O device 506.

Hereinafter, a method for calculating an optimized solution of multiple-mutated proteins using the computer **500** will be generally described.

A program (hereinafter referred to as an optimization program) which carries out the method of the present invention for calculating an optimum solution for the amino acid sequences of multiple-mutated proteins (Figure **1**) may be stored in the memory **502**, for example. Alternatively, the optimization program may be recorded in any recording medium, such as a floppy disk, MO, CD-ROM, and DVD-ROM. The optimization program recorded in such a recording medium is loaded through the I/O device **506** (e.g., a disk drive) to the memory **504** in the computer **500**. By executing the optimization program using the CPU **502**, the computer **500** functions as an apparatus which executes the method of the present invention for calculating an optimum solution for the amino acid sequences of multiple-mutated proteins.

The sequence data of a template protein population and the three-dimensional structure data and calculation parameters of the template protein population are input through the input section **501**.

The CPU **502** calculates a characteristic value of each member of the template protein population based on the information input through the input section **501**, and the characteristic value data is stored in the memory **504**. Thereafter, the CPU **502** applies a genetic algorithm to the template protein population based on the calculation parameters, the desired characteristic value, and the three-dimensional structure and characteristic value of the template protein population to produce a multiple-mutated protein population. Thereafter, the CPU **502** applies a dead end elimination algorithm to an amino acid side chain of an amino acid residue in each member of the multiple-mutated protein population, thereby optimizing the conformation of the amino acid side chain, and then executes energy minimization calculation. Thereafter, the CPU **502** calculates the three-dimensional structure data and characteristic value of each energy-minimized member in the multiple-mutated protein population, and stores the resultant three-dimensional structure data and characteristic value in the memory **504**.

Thereafter, the CPU **502** determines whether or not the above-described algorithm is repeated based on the calculation parameters, the characteristic value of each member of the template protein population and the characteristic value of each member of the multiple-mutated protein population. When it is determined that the above-described algorithm is repeated, the CPU **502** may further repeat the above-described algorithm.

In the case of repetition, the CPU **502** applies a genetic algorithm to the template protein population while taking into consideration the calculation parameters, the desired characteristic value and the characteristic value of the template protein population, and in addition, the characteristics which have been calculated to produce a multiple-mutated protein population. Subsequent processes are continuously carried out.

When the CPU **502** determines that the repetition is to be stopped, the CPU **502** selects a member having the desired characteristic value based on the characteristic value of each member in the template protein population and the characteristic value of each member in the multiple-mutated protein population stored in the memory **504**.

Thereafter, the output section **503** outputs the sequence data and characteristic value of the member selected by the CPU **502**. The output data may be output through the I/O device **506.**

Hereinafter, the method of the present invention will be described in detail with reference to Figure **1**.

The method of the present invention is a method for calculating an optimized solution of multiple-mutated proteins, and representatively comprises the following steps (10) to (50). Each step is executed by the input section **501,** the CPU **502,** or the output section **503** (Figure **6).**

Step 10: the sequence data of a template protein population and the three-dimensional structure data of the template protein population are input to the input section **501**.

In this step, the sequence data and the three-dimensional structure data of the template protein population used as basic data in the method of the present invention are input. The input data may be stored in the memory **504**. The sequence data may be an amino acid sequence or a nucleic acid sequence. The amino acid sequence may be modified with a modifying group (e.g., a sugar chain, fatty acid, sulfate groups, and the like). Amino acids used in an amino acid sequence may be either or both naturally occurring amino acids or non-naturally occurring amino acids. Data of amino acid sequences or nucleic acid sequences may be obtained from a known database (SwissProt, Gen-Bank, or the like), or may be newly determined by a well-known technique in the art (e.g., Sanger method, Edman method, and the like). The input three-dimensional structure data may be atomic coordinate data, for example. Atomic coordinates may be, for example, experimental data from X-ray structural analysis or the like or coordinate data produced by modeling, calculation, or the like. The three-dimensional structure data may be obtained from a known database (e.g., PDB or the like), for example.

Step 12: the CPU **502** calculates the characteristic value of each member in the template protein population based on the sequence data and the three-dimensional structure data of the above-described template protein population. The calculated data may be stored in the memory **504**. Based on the data input in step 10, the characteristic value to be used in the method of the present invention is calculated. The characteristic value is a determining factor in determining the optimum value. Examples of the characteristic value which may be used in the present invention include empirical molecular mechanics potential, semi-empirical quantum mechanics potential, non-empirical quantum mechanics potential, electromagnetic potential, and solvation potential and structural entropy.

Step 14: calculation parameters and desired characteristic values, which are used in executing the algorithm described below, are input to the input section **501**.

In this step, calculation parameters and the like, which are used in executing an algorithm in the method of the present invention, are input. The input data may be stored in the memory **504**. Examples of the calculation parameters to be input include parameters in a genetic algorithm, such as the number of generations, a mutation rate, a selection rate, a selection method, a crossover rate, a crossover method, and the like. The calculation parameters may be characteristic values which are criteria for selection. The calculation parameters may also be evaluations of generations, such as a condition in which the optimum value among N-1 generations is equal to the optimum value among N generations.

The desired characteristic value can be any characteristic value of a multiple-mutated protein aimed to be obtained by the method of the present invention. Examples of the desired characteristic value include empirical molecular mechanics potential, semi-empirical quantum mechanics potential, non-empirical quantum mechanics potential, electromagnetic potential, solvation potential, structural entropy, and the like. The desired characteristic value may be a biochemical characteristic value of a protein. Alternatively, the desired characteristic value may be related directly or indirectly to a biochemical characteristic value of a protein. Therefore, the desired characteristic value may be changed depending on a mutation in a biochemical characteristic of a protein.

It is clear that those skilled in the art can appropriately set calculation parameters depending on the desired characteristic value.

Step 20: the CPU 502 applies a genetic algorithm to the above-described template protein population based the above-described calculation parameters, the desired characteristic value, and the characteristic value of the above-described template protein population to produce a multiple-mutated protein population.

In this step, a genetic algorithm is applied to the template protein population. As described above, in the genetic algorithm, the input population is subjected to evaluation of individuals, selection, reproduction, crossover, mutation, and evaluation of groups. All of the selection, the reproduction, the crossover and the mutation may be carried out or at least one of them may not be carried out. Step 20 is a first application of the genetic algorithm to the input template protein population. In the first application of the genetic algorithm, the mutation rate is preferably high (e.g., 50%, 75%, 100%, or the like) in order to prevent the genetic algorithm from falling into a local minimum so that a sufficient level of diversity is secured, if necessary. Data produced in this step is stored in the memory **504.**

Step 22: the CPU **502** applies a dead end elimination algorithm to the amino acid side chains of the amino acid residues of each member in the above-described multiple-mutated protein population to optimize the conformation of the above-described amino acid side chain, and thereafter, carries out energy minimization calculation.

In this step, the atomic coordinates of each amino acid residue in the amino acid sequence of each member in the multiple-mutated protein population produced in step 20 are optimized by the dead end elimination algorithm. Thereafter, energy minimization is carried out. In the dead end elimination algorithm, all amino acid residues may be processed, or alternatively, a part or all of the amino acid residues which are not mutated may be fixed. Preferably, the dead end elimination algorithm may be applied to mutated amino acid residues and their surrounding non-mutated amino acid residues. Data produced in this step may be stored in the memory **504**, or may be output through the output section **503**. Here, the output data may be names uniquely indicating atoms constituting a protein and the structural coordinates of these atoms.

Step 24: the CPU **502** calculates the three-dimensional structure data and the characteristic value of each member, whose energy is minimized, in the above-described multiple-mutated protein population.

In this step, the three-dimensional structure data of the above-described protein population which has been subjected to energy minimization is calculated by the above-described well-known method or the like, and the characteristic value is calculated by a method similar to that carried out in step 12. The calculated data are candidates for solutions, and are stored in a storage section if necessary. Data produced in this step may be stored in the memory **504**.

Step 30: the CPU **502** determines whether or not the following steps 21, 23 and 25 are to be carried out based on the above-described calculation parameters, the above-described desired characteristic value, the characteristic value of each member in the above-described template protein population, and the characteristic value of each member in the above-described multiple-mutated protein population.

The characteristic values of the multiple-mutated protein population calculated in steps 20, 22 and 24 are evaluated to determine whether or not the desired characteristic value is obtained, or whether or not a genetic algorithm is to be applied again based on any of the calculation parameters for the genetic algorithm. The determination in this step may be carried out based on the number of times. In this case, for example, the repetition may be stopped after the $N^{th}$ time, where N is the number of times at which the optimum value of the characteristic value of a protein which has been calculated N-1 times first becomes equal to that resulting from the $N^{th}$ calculation. When it is determined that steps 21, 23 and 25 are not to be carried out, the process goes to step 40.

Step 21: when in step 30 it is determined that steps 21, 23 and 25 are to be carried out, or that the genetic algorithm is to be repeated in step 31 described below, the CPU **502** applies the genetic algorithm to the above-described template protein population based on the above-described calculation parameters, the above-described desired characteristic value and the characteristic value of the above-described template protein population, and the characteristic value of each of all members, which have been produced, in the multiple-mutated protein population to produce a new multiple-mutated protein population. Data produced in this step may be stored in the memory **504**.

In this step, a genetic algorithm is applied to a population including the template protein population and the produced multiple-mutated protein population. In the genetic algorithm, as described above, the input population is subjected to evaluation for individuals, selection, reproduction, crossover, mutation, and evaluation of groups. All of the selection, the reproduction, the crossover and the mutation may be carried out or at least one of them may not be carried out. Step 21 is a second application of the genetic algorithm. In the second application and thereafter of the genetic algorithm, the genetic algorithm may be applied to the protein members in the multiple-mutated protein population which have been generated by the genetic algorithm as well as the protein members in the template protein population. The mutation rate is preferably high (e.g., 50%, 75%, 100%, or the like) in order to prevent the genetic algorithm from falling into a local minimum so that a sufficient level of diversity is secured, if necessary. Data produced in this step is stored in the memory **504**.

Step 23: the CPU 502 applies a dead end elimination algorithm to the amino acid side chains of the amino acid residues of each member in the above-described multiple-mutated protein population to optimize the conformation of the above-described amino acid side chain, and thereafter, carries out energy minimization calculations. Data produced in this step is stored in the memory **504**.

In this step, the position of each amino acid residue in the amino acid sequence of each member in the multiple-mutated protein population produced in step 21 are optimized by the dead end elimination algorithm. Thereafter, energy minimization is carried out. It should be noted that minimization is omitted for protein members which have already been subjected to minimization. In the dead end elimination algorithm, all amino acid residues may be processed, or alternatively, amino acid residues which have not been mutated may be fixed.

Step 25: the CPU **502** calculates the three-dimensional structure data and the characteristic value of each member, whose energy is minimized, in the above-described multiple-mutated protein population.

In this step, the three-dimensional structure data of the above-described protein population which has been subjected to energy minimization is calculated by a well-known method in the art, and the characteristic value of each member in the protein population is calculated by a method similar to that carried out in step 12. The calculated data are candidates for solutions, and may be stored in the memory **504.**

Step 31: the CPU **502** determines whether or not the following steps 21, 23 and 25 are carried out based on the above-described calculation parameters, the above-described desired characteristic value, the characteristic value of each member in the above-described template protein population, and the characteristic value of each member in the above-described multiple-mutated protein population. The determination in this step may be carried out based on the number of times. In this case, for example, the repetition may be stopped after the N$^{th}$ time, where N is the number of times at which the optimum value of the characteristic value of a protein which has been calculated N-1 times first becomes equal to that resulting from the N$^{th}$ calculation. When it is determined that steps 21, 23 and 25 are not carried out, the process goes to step 40.

Step 40: the CPU **502** selects a member having the above-described desired characteristic value from the characteristic value of each member in the above-described template protein population and the characteristic value of each of all members, which have been produced, in the multiple-mutated protein population.

After the production of the multiple-mutated protein population has ended, in this step, the characteristic values of the protein members which have been produced are compared with each other to select a protein member having the desired characteristic value. Such a member may be selected from the data stored in the memory **504**. The number of selected members may be one or more, for example, at least 5, 10, 20, 50, 100 or 200. In some cases, a member having the desired characteristic value may be selected from the members in the template protein population. Typically, however, a member having the desired characteristic value is selected from the members in the multiple-mutated protein population. It should be noted that individuals having the desired characteristic value do not necessarily occupy a large portion of the population.

Step 50: the output section **503** outputs the sequence data and the characteristic value of the selected member. In this step, the sequence data and the characteristic value of a protein member having the desired characteristic value, which has been selected in step 40. Any output form may be employed. For example, a list of ranking in terms of characteristic values from the optimum value may be used. The data may be printed out on paper, or may be stored in a storage medium (e.g., a magnetic storage device (e.g., a hard disk, a floppy disk, and the like), an optical storage device (e.g., a MO disk and the like), and the like).

It should be noted that in the example shown in Figure **6**, each section included in the apparatus for calculating an optimized solution of multiple-mutated proteins is implemented by software. Therefore, the present invention also relates to a program for causing a computer to execute the method of the present invention. Such a computer program may be produced by a well-known technique in the art. The function of each section of the apparatus for calculating an optimized solution of multiple-mutated proteins can be implemented by hardware (circuits).

Next, a genetic algorithm which is applied to the present invention will be described.

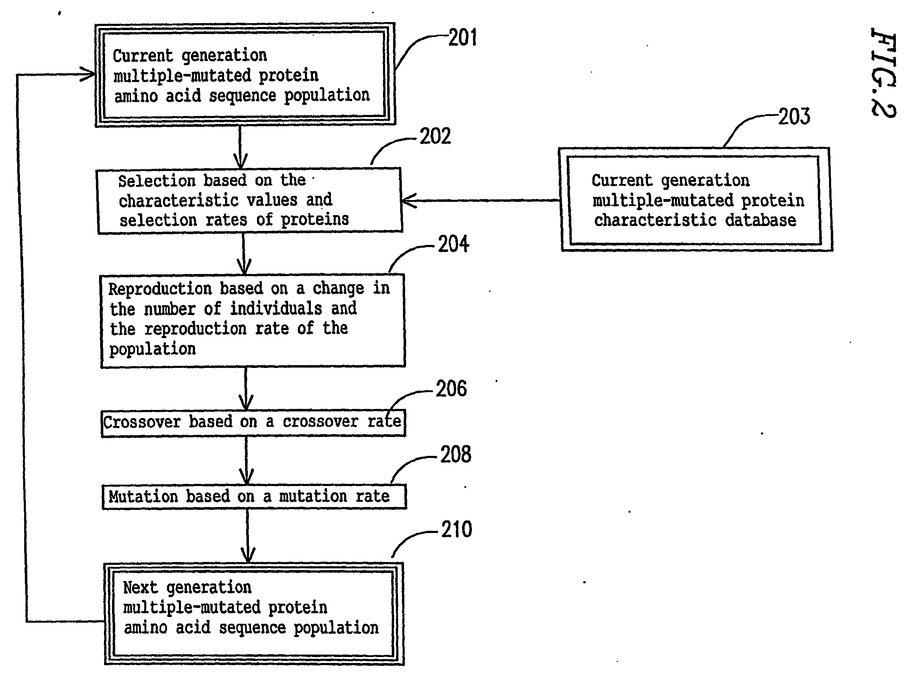

Figure **2** shows a scheme of a GA for one generation. A GA process is carried out for a multiple-mutated protein amino acid sequence population **(201)** in a current generation. In the present invention, the GA process is carried out by a combination of: a process **(202)** in which selection is carried out based on the characteristic values and selection rates of proteins obtained from a multiple-mutated protein characteristic value database (**203**) for the current generation; a process (**204**) in which reproduction is carried out based on a change in the number of individuals and the reproduction rate of the population; a process (206)in which crossover is carried out based on a crossover rate; and a process (208) in which mutation is carried out based on a mutation rate. These processes are successively carried out, so that a multiple-mutated protein amino acid sequence population **(210)** for a next generation is obtained. The order of the processes **(202)** to **(208)** to be carried out may be different from that shown in Figure 2. Any of the processes may not be carried out. Variables used in GA in the processes **(202)** to **(208),** i.e., the number of individuals in a population, the number of generations, a selection rate, a reproduction rate, a crossover rate and a mutation rate, can be changed for each generation and each population, and the types of amino acids which are permitted to be mutated can be limited.

Hereinafter, a dead end elimination (DEE) algorithm which is applied to the present invention will be described.

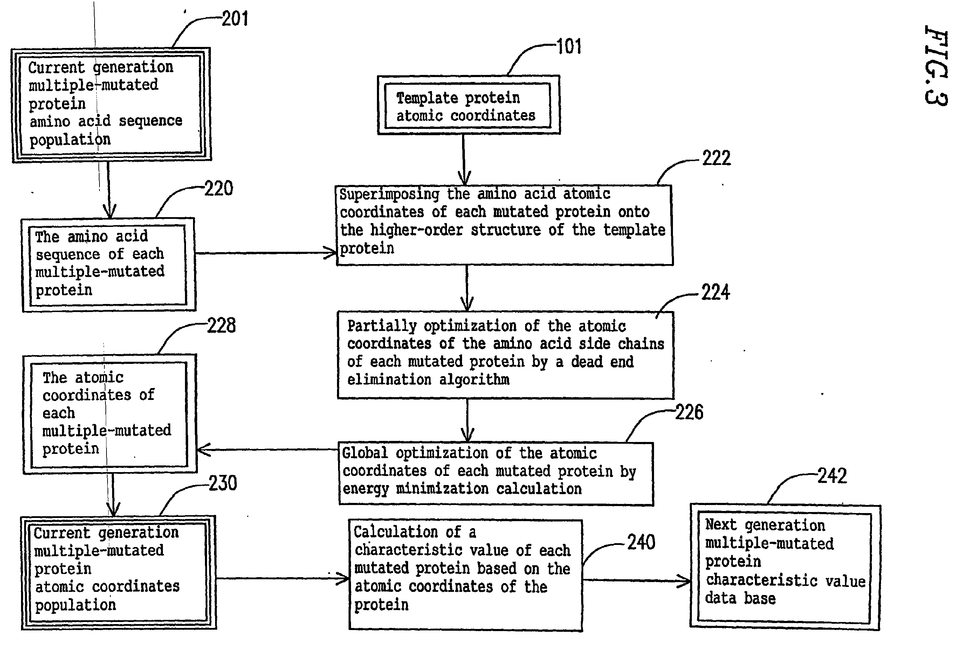

Figure **3** shows a process for calculating the three-dimensional structure atomic coordinates of a mutated protein population in one GA generation, and then calculating the characteristic value of each protein. Initially, a multiple-mutated protein amino acid sequence (**220**) is successively selected from a multiple-mutated protein amino acid sequence population (**201**) in a current generation. Based on information on the amino acid sequence, a temporary mutated protein amino acid atomic coordinates are superimposed onto template protein three-dimensional structure atomic coordinates **(101) (222).** This temporary atomic coordinates are subjected to a dead end elimination algorithm so as to partially optimize the amino acid side chain atomic coordinates of mutated proteins **(224).** Further, energy minimization calculation is carried out so as to globally optimize the amino acid side chain atomic coordinates of mutated proteins (**226**). Thus, the optimized multiple-mutated protein atomic coordinates (**228**) are obtained. The process **(222)** to **(226)** are successively carried out, thereby obtaining a multiple-mutated protein atomic coordinates population **(230)** for the current generation. These protein atomic coordinates are used to calculate the characteristic value of each protein (**240**), thereby producing a multiple-mutated protein characteristic value database (**242**) for the current generation. This characteristic value database may be used as calculation parameters in GA.

In general, if a protein amino acid sequence is multiple-mutated, each amino acid mutation has an additive effect on the characteristic of a protein. However, it is known in rare cases, particular multiple amino acid mutations have a non-additive effect. Therefore, in order to design a mutated protein having a desired characteristic efficiently, a process of combining amino acid mutations which add a desired characteristic to a mutated protein, and a process of considering multiple amino acid mutations having a non-additive effect need to be combined. GA, a global optimization method, has a search characteristic such that the above-described additive amino acid mutations and non-additive multiple amino acid mutations are simultaneously taken into consideration, thereby making it possible to optimize the amino acid sequences of multiple-mutated proteins.

With GA, the protein three-dimensional structure atomic coordinates and the protein characteristic values of all multiple-mutated protein amino acid sequences (all candidates for solutions) are not calculated. Only for a portion of the candidates for solutions, the protein three-dimensional structure atomic coordinates and the protein characteristic values are calculated, thereby obtaining the optimum solution, and significantly reducing the calculation time without a decrease in the calculation accuracy.

In the method of the present invention for calculating the optimum solution for multiple-mutated protein amino acid sequences, DEE calculation is carried out for the amino acid side chain three-dimensional structure of multiple-mutated protein amino acid sequences (i.e., candidates for solutions) under a constraint condition that a template protein high-order structure is generally maintained. Thereafter, energy minimization calculation is carried out, thereby obtaining the three-dimensional structure atomic coordinates of multiple-mutated proteins with good accuracy. The three-dimensional structure atomic coordinates of a multiple-mutated proteins (candidates for solutions) are often unknown. Moreover, a large amount of resource are expended to newly determine atomic coordinates by experimentation. Therefore, it is useful to use the above-described method to obtain atomic coordinates with good accuracy without calculating all candidates.

The resultant three-dimensional structure atomic coordinates of multiple-mutated proteins can be used to calculate the characteristic values of useful proteins with good accuracy. The characteristic value of a protein obtained from the amino acid sequences of the multiple-mutated proteins is usually limited. In often cases, the characteristic value is not obtained with high accuracy. With three-dimensional structure atomic coordinates, for example, the molecular mechanics potential or quantum mechanics potential of mutated proteins can be calculated to obtain variations in free energy in the course of thermal denaturation of the mutated proteins. Based on the variations, the thermal or chemical stability of a protein, or further the strength of a bond between the protein and other molecules can be calculated.

When mutating amino acid sequences with a GA, the number of populations, the number of individuals in each population, the number of generations, a selection rate, a reproduction rate, a crossover rate, and a mutation rate can be changed to optimize a multiple-mutated protein amino acid sequence depending on desired design parameters. For example, the number of individuals in each population and a crossover rate or a mutation rate can be appropriately designed to control the magnitude of the difference between the amino acid sequence of a template protein and the multiple-mutated amino acid sequences of candidates for solutions, thereby making it possible to cause an optimized mutation to be close to or far from the template in a selective manner.

When mutating amino acid sequences with a GA, the number of populations and the types of amino acids which are permitted to be mutated can be limited to optimize a multiple-mutated protein amino acid sequence depending on desired design factors. For example, an amino acid type at a particular amino acid mutation site can be limited to a basic amino acid type, an acidic amino acid type, or the like to optimize the thermal stability of a mutated protein without deviating the electrostatic characteristic of the multiple-mutated protein from that of a template protein.

(Transmission medium)

The present invention also provides a transmission medium for transmitting the program of the present invention. The term "transmission" as used herein refers to sending of data from one place to another. The term "transmission medium" refers to a medium which transmits information, such as a program, data (e.g., news, contents, and the like), and the like by a method, such as cable, wireless, and the like. Such transmission media are well-known to those skilled in the art. Examples of such transmission media include communication media, such as optic fibers, cables, wireless systems, and the like. Such communication media are used to construct a computer network system, such as LAN, the Internet, intranet, WAN (e.g., extranet), wireless communication network. Such networks include broadcast networks and communication networks. Transmission media of the present invention achieve the effects of the present invention by transmitting the program of the present invention through a network as described above. Such an effect cannot be achieved by transmission media for transmitting conventional programs. Therefore, the transmission media of the present invention have an unexpected advantageous effect over conventional transmission media.

(Business-related method)

The present invention also relates to a method for providing a service using the method of the present invention. More specifically, the present invention relates to a method for providing a service for calculation of an optimized solution of the amino acid sequences of multiple-mutated proteins based on input data over a network.

A method for providing a service using the method of the present invention may be carried out through a transmission medium as described above. Therefore, the method for providing a service using the method of the present invention includes providing a service to customers through a leased line, and providing a service to customers extensively over the Internet. Such a service may be provided by electronic mail or on a web site (WWW). Cryptography may be used to provide a service.

The present invention may be implemented as a process on a computer by installing a server on the service provider side.

Therefore, the method of the present invention comprises:

the step of inputting the three-dimensional structure data, the amino acid sequence data and the calculation parameters of a template protein population to a server over a network or another means, and

the step of the server calculating the optimum three-dimensional structural coordinates for members of a multiple-mutated protein population by carrying out a dead end elimination algorithm to search the three-dimensional structural coordinates of amino acid side chains of the amino acid sequences of each member and carrying out energy minimization calculations for the structure of the members, based on the three-dimensional structure data of a template protein population;

the step of the server calculating a characteristic value from the optimum three-dimensional structural coordinates of a multiple-mutated protein; and

the step of the server calculating a member which optimizes the characteristic value of the multiple-mutated protein population by using a genetic algorithm.

Information or data (e.g., the sequence data of a template protein population, the three-dimensional structure data of the template protein population, calculation parameters and desired characteristic values used in carrying out the algorithm, and the like) used in the present invention is input by a service receiver over the Internet or the like to a server possessed by a provider on the Internet. Such a server may comprise a database for storing the input data.

The input data may be stored in a volatile memory or a non-volatile memory. This server may contain the program of the present invention. Such a program may be recorded in a recording medium, such as a hard disk and the like, which may be installed in the server. Such a program may also be recorded in any type of recording medium, such as a floppy disk, MO, CD-ROM, and DVD-ROM. The program of the present invention recorded in such a recording medium is loaded, for example, through the I/O device **506** (e.g., a disk drive) shown in Figure **6** to the memory **504** in the computer **500**. With the CPU **502** executing an optimization program, the computer **500** functions as a server which carries out the method of the present invention for calculating an optimum solution for multiple-mutated protein amino acid sequences.

Further, such a server may be connected through a network node to a network, such as the Internet. With the server connected to the network, the present invention can provide a service for calculating an optimized solution of the amino acid sequences of multiple-mutated proteins based on data input over the network.

In another preferred embodiment of the present invention, the method of the present invention comprises:

(a) the step of inputting the sequence data and the three-dimensional structure data of a template protein population over a network, and

(b) the step of a server calculating the characteristic value of each member in the template protein population based on the sequence data and the three-dimensional structure data of the template protein population;

(c) the step of inputting calculation parameters and a desired characteristic value to be used in the above-described algorithm over the network;

(d) the step of the server applying a genetic algorithm to the template protein population to generate a multiple-mutated protein population based on the calculation parameters, the desired characteristic value and the three-dimensional structure data and the characteristic value of each member in the template protein population;

(e) the step of the server applying a dead end elimination algorithm to the amino acid side chains of the amino acid residues of each member in the multiple-mutated protein population to optimize the conformations of the amino acid side chains, and carrying out energy minimization calculations;

(f) the step of the server calculating the three-dimensional structure data and the characteristic value of each member having a minimized energy in the multiple-mutated protein population;

(g) the step of the server determining whether or not steps (h) to (j) are to be carried out based on the calculation parameters, the desired characteristic value, the three-dimensional structure data and the characteristic value of each member in the template protein population, and the three-dimensional structure data and the characteristic value of each member in the multiple-mutated protein population;

(h) the step of the server, when the server determines that step (g) is executed, generating a new multiple-mutated protein population based on the calculation parameters, the desired characteristic value and the characteristic value of the template protein population, and the characteristic value of each member in the multiple-mutated protein populations, which have been generated, by applying a genetic algorithm to the template protein population;

(i) the step of the server applying the dead end elimination algorithm to the amino acid side chains of the amino acid residues of each member in the new multiple-mutated protein population to optimize the conformations of the amino acid side chains, and carrying out energy minimization calculation;

(j) the step of the server calculating the three-dimensional structure data and the characteristic value of each member having a minimized energy in the new multiple-mutated protein population;

(k) the step of the server determining whether or not steps (h) to (j) are to be carried out based on the calculation parameters, the desired characteristic value, the characteristic value of the template protein population, and the characteristic value of each member in all of the multiple-mutated protein populations which have been generated;

(l) the step of the server selecting a member having the desired characteristic value from the characteristic value of each member in the template protein population and the characteristic value of each member in all of the multiple-mutated protein populations which have been generated; and

(m) the step of the server outputting the sequence data and the characteristic value of the selected member.

Hereinafter, an example of the present invention will be illustrated. It is understood by those skilled in the art that such an example is provided only for the purpose of explaining the present invention and is not intended to restrict the scope or gist of the present invention. Thus, the present invention is defined only by the claims attached hereto. Therefore, those skilled in the art can practice the present invention by arbitrarily modifying the example within the scope of the attached claims with reference to the example.

Example

An example will be shown, in which an attempt was made to design a λ-repressor protein, which is one of regulators in DNA transcription, in such a manner as to improve the heat resistance thereof. The three-dimensional structure atomic coordinates of a wild-type λ-repressor protein of E. coli are registered and published in the Protein Data Bank (registration number: 1LMB). The atomic coordinates were used as a template protein.

Valine 36, methionine 40 and valine 47 amino acid residues of a wild-type λ-repressor are located in a so-called hydrophobic core thereof. By multiple-mutating these three residues, it was expected to design a mutated λ-repressor protein which is more heat resistant than the wild-type one.

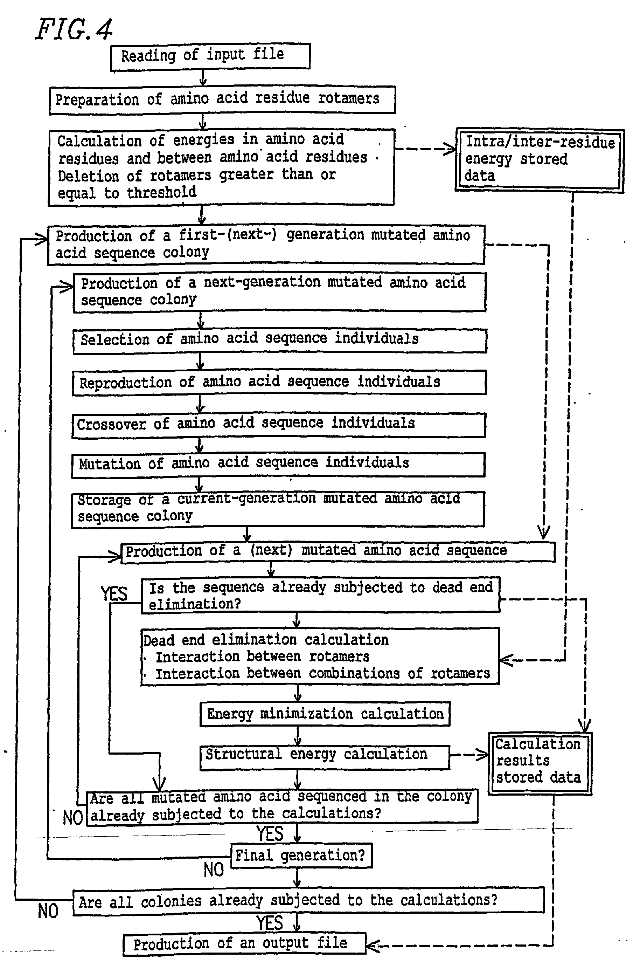

In actual multiple mutation calculation, the computer program shrike (available from Kaneka Corporation) in which the algorithm of the present invention was implemented was used to design the above-described improvement of heat resistance. An exemplary arrangement of this implementation is shown in Figure **4.**

As for calculation parameters in a GA process, the number of calculations, i.e., the number of populations was 2, the number of members in a mutated protein population, i.e., the number of individuals was 100, the number of generations was 40, a mutation rate was 100% only for the initial time and 20% thereafter, a selection rate, i.e., a survival rate was 70%, a crossover rate was 20%, and a reproduction rate was constant irrespective of the number of individuals. As a desired characteristic value for optimization of the three-dimensional structure of mutated proteins, AMBER molecular force field potential and solvation potential were used. As a characteristic value for calculation of a characteristic of a protein, AMBER molecular force field potential and solvation potential were used to calculate the overall structural energy of the protein, and the resultant energy value was used as a characteristic value of the protein. The difference in a structural energy value between two different members in the multiple-mutated protein population structural energy was used as an index of the thermal stability of the two mutated proteins. As a constraint for the type of an amino acid to be mutated, the amino acid could be mutated to any of the 20 naturally occurring amino acids.

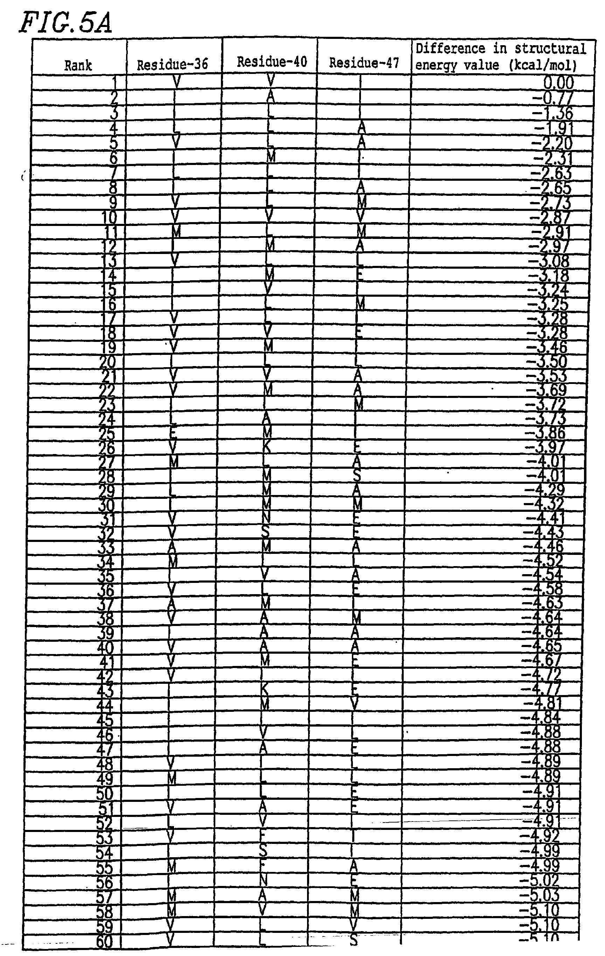

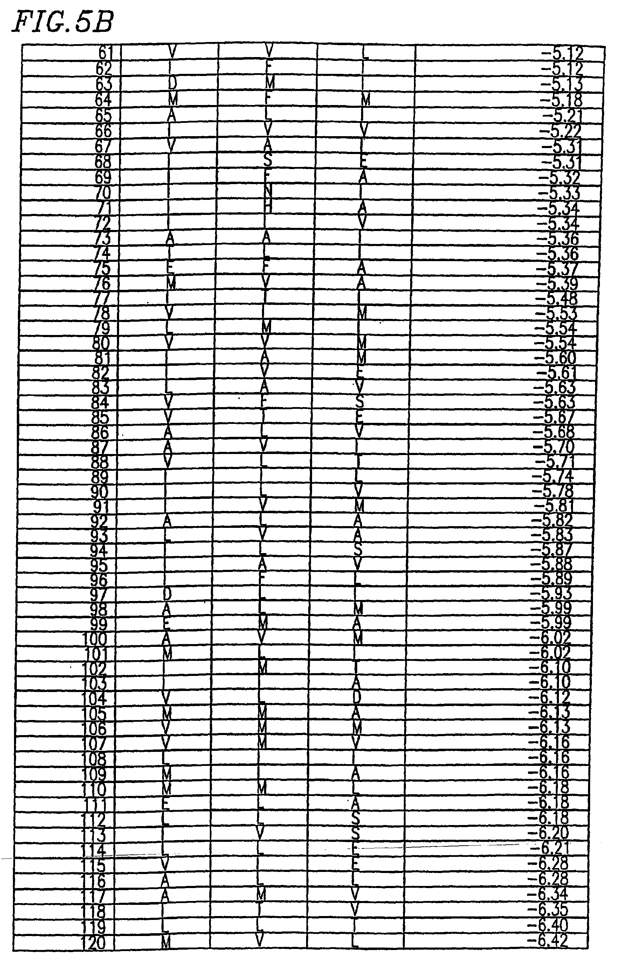

All multiple-mutated protein amino acid sequences obtained by the GA process were ranked using the above-described structural energy values as indicators (design result). For 120 highly-ranked multiple-mutated proteins in this result, amino acids at positions 36, 40 and 47 in the mutated amino acid sequences are shown in Figures **5A** and **5B** using one-character codes. In addition, the protein characteristic values, i.e., the structural energy difference value of each mutated amino acid sequence are shown in Figures **5A** and **5B.**

The calculation and design results shown in Figures **5A** and **5B** and the experimental results of synthesized mutated proteins with available data are compared with each other. The result is shown below. Specifically, according to experimental results described in Journal of Molecular Biology (1991) vol. 219 359-376, a seventh-ranked mutated protein (Leu-36, Leu-40, Ile-47) and a fourty-fourth-ranked mutated protein (Ile-36, Met-40, Val-47) shown in Figures **5A** and **5B,** are more heat resistant than a 107th-ranked wild-type protein (Val-36, Met-40, Val-47) shown in Figures **5A** and **5B.**

Table 1

| Core sequence of mutant | Melting point of protein structure | Ranking based on calculation results in the present invention |
|---|---|---|
| Leu-36, Leu-40, Ile-47 | 60°C | 7 |
| Ile-36, Met-40, Val-47 | 59°C | 44 |
| Val-36, Met-40, Val-47 (wild type) | 56°C | 107 |

According to this result, the mutated protein design means of the present invention could be used to select a mutated protein design proposal. in which the characteristic value of interest can be optimized, without reducing accuracy.

The total number of amino acids of mutated proteins output as results in this example was 516. The calculation time in the example was 3.6 hours where Orlgin200 (SGI) was used as a computer. On the other hand, when the GA process shown in the present invention was not used and all possible combinations of amino acid sequences were calculated, i.e., 20 naturally occurring amino acid mutations were carried out at each of three mutation sites (i.e., a total of 8000), the calculation time was 31.4 hours where the above-described computer was used.

The 200 highly-ranked output results in the present invention were compared with 200 highly-ranked output results by a conventional method. 198 output results were shared by both. Therefore, for the 200 highly-ranked output results, it is clear that a calculation accuracy of 99% was obtained.

Thus, it is possible to reduce a calculation time by a factor of about 10 by employing the method of the present invention without decreasing calculation accuracy.

According to this result, the mutated protein design means of the present invention could be used to select a mutated protein design proposal, in which the characteristic value of interest can be optimized, within a short time. Further, according to the method of the present invention, an optimum solution approximate to that obtained by molecular evolution in the nature can be obtained, which cannot be conventionally predicted and is not achieved by a protein design technique using a DEE algorithm alone (Malakauskas, S. et al. (1998), Nature Structural Biology, 5, 470-475).

(Effects of the Invention)

It is possible to obtain an optimum solution conforming with natural conditions for mutated proteins at high speed and without decreasing accuracy.

INDUSTRIAL APPLICABILITY

According to the present invention, it is possible to select an optimum solution from a multiple-mutated protein population having an enormous number of combinations, based on a characteristic value, without decreasing accuracy and within a short time. Particularly, the present invention provides a method and apparatus for modifying any one or a combination of the thermal stability, the chemical stability, the chemical selectivity to a substrate, the stereoselectivity to a substrate, and the optimal pH value of an industrially useful enzyme or a signal transduction protein, and a storage medium carrying a program which describes such a method.

**Claims**

1. A method for calculating an optimized solution of the amino acid sequences of multiple-mutated proteins, comprising the steps of:

   searching the three-dimensional structural coordinates of amino acid side chains of the amino acid sequences of members of amultiple-mutated protein population based on the three-dimensional structure data of a template protein population using a dead end elimination algorithm, and executing structural energy minimization calculations for the members, thereby calculating the three-dimensional structural coordinates of an optimum multiple-mutated protein;
   calculating a characteristic value from the three-dimensional structural coordinates of the optimum multiple-mutated protein; and
   applying a genetic algorithm to the multiple-mutated protein population to calculate the members which optimize the characteristic value.

2. A method according to claim 1, wherein the step of calculating the three-dimensional structural coordinates of the optimum multiple-mutated protein is carried out under a constraint that the three-dimensional structure of the template protein is generally maintained.

3. A method for calculating an optimized solution of the amino acid sequences of multiple-mutated proteins, comprising the steps of:

   (a) inputting sequence data and three-dimensional structure data of a template protein population;
   (b) calculating a characteristic value of each member in the template protein population based on the sequence data and the three-dimensional structure data of the template protein population;
   (c) inputting calculation parameters and a desired characteristic value to be used in the algorithm;
   (d) applying a genetic algorithm to the template protein population to generate a multiple-mutated protein population based on the calculation parameters, the desired characteristic value and the three-dimensional structure data and the characteristic value of each member in the template protein population;
   (e) applying a dead end elimination algorithm to amino acid side chains of amino acid residues of each member in the multiple-mutated protein population to optimize the conformations of the amino acid side chains, and carrying out energy minimization calculations;
   (f) calculating three-dimensional structure data and characteristic value of each member having a minimized energy in the multiple-mutated protein population;
   (g) determining whether or not steps (h) to (j) are to be carried out based on the calculation parameters, the desired characteristic value, the three-dimensional structure data and the characteristic value of each member in the template protein population, and the three-dimensional structure data and the characteristic value of each member in the multiple-mutated protein population;
   (h) when in step (g) it is determined that steps (h) to (j) are carried out, applying a genetic algorithm to the template protein population to generate a new multiple-mutated protein population based on the calculation parameters, the desired characteristic value and the characteristic value of the template protein population,

22

and the characteristic value of each member in the multiple-mutated protein populations which have been generated;

(i) applying the dead end elimination algorithm to amino acid side chains of amino acid residues of each member in the new multiple-mutated protein population to optimize the conformations of the amino acid side chains, and carrying out energy minimization calculations;

(j) calculating three-dimensional structure data and a characteristic value of each member having a minimized energy in the new multiple-mutated protein population;

(k) determining whether or not steps (h) to (j) are carried out based on the calculation parameters, the desired characteristic value, the characteristic value of the template protein population, and the characteristic value of each member in all of the multiple-mutated protein populations which have been generated;

(l) selecting a member having the desired characteristic value from the characteristic values of the members in the template protein population and the characteristic values of the members in all of the multiple-mutated protein populations which have been generated; and

(m) outputting the sequence data and the characteristic value of the selected member.

4. A method according to claim 1 or 3, wherein the sequence data of the template protein population is of amino acid sequence and/or nucleic acid sequence.

5. A method according to claim 1 or 3, wherein the three-dimensional structure data of the template protein population includes at least one selected from the group consisting of atomic coordinate data, molecular topology data, and molecular force field constants.

6. A method according to claim 1 or 3, wherein the template protein population includes one member.

7. A method according to claim 1 or 3, wherein the template protein population includes at least two members.

8. A method according to claim 1 or 3, wherein the characteristic value or the desired characteristic value includes at least one data selected from the group consisting of empirical molecular mechanics potential, semi-empirical quantum mechanics potential, non-empirical quantum mechanics potential, electromagnetic potential, and solvation potential and structural entropy.

9. A method according to claim 3, wherein the calculation parameters are calculation parameters for the genetic algorithm.

10. A method according to claim 3, wherein the calculation parameters include a characteristic value which is a criterion for the determination in step (g).

11. A method according to claim 3, wherein the calculation parameters include information for specifying the conformations of amino acids to be mutated.

12. A method according to claim 1 or 3, wherein the dead end elimination algorithm is applied to at least one of the amino acid residues.

13. A method according to claim 1 or 3, wherein the dead end elimination algorithm is applied to all of the amino acid residues.

14. A method according to claim 1 or 3, wherein a protein characteristic to be modified is selected from thermal stability, chemical stability, chemical selectivity to a substrate, stereoselectivity to a substrate, and optimal pH value.

15. A method according to claim 4, wherein the amino acid sequence is selected from the group consisting of naturally occurring amino acids, chemically modified amino acids, and non-naturally occurring amino acids.

16. A method according to claim 1 or 3, wherein each member of the multiple-mutated protein population is a molecular complex including at least one protein comprising a plurality of homologous molecules, a plurality of heterologous molecules, or a combination thereof.

17. An apparatus for calculating an optimized solution of the amino acid sequences of multiple-mutated proteins, comprising:

means for searching the three-dimensional structural coordinates of amino acid side chains of the amino acid sequences of members of a multiple-mutated protein population based on the three-dimensional structure data of a template protein population using a dead end elimination algorithm, and executing structural energy minimization calculations for the members, thereby calculating the three-dimensional structural coordinates of an optimum multiple-mutated protein;

means for calculating a characteristic value from the three-dimensional structural coordinates of the optimum multiple-mutated protein; and

means for applying a genetic algorithm to the multiple-mutated protein population to calculate the members which optimize the characteristic value.

18. A method according to claim 17, wherein the means for calculating the three-dimensional structural coordinates of the optimum multiple-mutated protein is carried out under a constraint that the three-dimensional structure of the template protein is generally maintained.

19. An apparatus for calculating an optimized solution of the amino acid sequences of multiple-mutated proteins, comprising:

(1) an input section;
(2) a calculation section; and
(3) an output section,

wherein the input section comprises:

(a) means for inputting sequence data and three-dimensional structure data of a template protein population; and
(b) means for inputting calculation parameters and a desired characteristic value to be used in the algorithm,

the calculation section "comprises :

(c) means for calculating a characteristic value of each member in the template protein population based on the sequence data and the three-dimensional structure data of the template protein population,
(d) means for applying a genetic algorithm to the template protein population to generate a multiple-mutated protein population based on the calculation parameters, the desired characteristic value and the three-dimensional structure data and the characteristic value of each member in the template protein population;
(e) means for applying a dead end elimination algorithm to amino acid side chains of amino acid residues of each member in the multiple-mutated protein population to optimize the conformations of the amino acid side chains, and carrying out energy minimization calculations;
(f) means for calculating three-dimensional structure data and characteristic value of each member having a minimized energy in the multiple-mutatedprotein population, and storing the calculated three-dimensional structure data and characteristic value;
(g) means for determining whether or not the steps for generating a population carried out by the means (d) to (f) are to be carried out based on the calculation parameters, the desired characteristic value, the three-dimensional structure data and the characteristic value of each member in the template protein population, and the three-dimensional structure data and the characteristic value of each member in the multiple-mutated protein population; and
(i) selecting a member having the desired characteristic value from the characteristic values of the members in the template protein population and the characteristic values of the members of the multiple-mutated protein populations,

wherein the output section comprises:

means for outputting the sequence data and characteristic value of the selected member.

20. An apparatus according to claim 17 or 19, wherein the sequence data of the template protein population is of amino acid sequence and/or nucleic acid sequence.

21. An apparatus according to claim 17 or 19, wherein the three-dimensional structure data of the template protein population includes at least one selected from the group consisting of atomic coordinate data, molecular topology data, and molecular force field constants.

22. An apparatus according to claim 17 or 19, wherein the template protein population includes one member.

23. An apparatus according to claim 17 or 19, wherein the template protein population includes at least two members.

24. An apparatus according to claim 17 or 19, wherein the characteristic value or the desired characteristic value includes at least one data selected from the group consisting of empirical molecular mechanics potential, semi-empirical quantum mechanics potential, non-empirical quantum mechanics potential, electromagnetic potential, and solvation potential and structural entropy.

25. An apparatus according to claim 19. wherein the calculation parameters are calculation parameters for the genetic algorithm.

26. An apparatus according to claim 19, wherein the calculation parameters include a characteristic value which is a criterion for the determination in step (g).

27. An apparatus according to claim 19, wherein the calculation parameters include information for specifying the conformations of amino acids to be mutated.

28. An apparatus according to claim 17 or 19, wherein the dead end elimination algorithm is applied to at least one of the amino acid residues.

29. An apparatus according to claim 17 or 19, wherein the dead end elimination algorithm is applied to all of the amino acid residues.

30. An apparatus according to claim 17 or 19, wherein a protein characteristic to be modified is selected from thermal stability, chemical stability, chemical selectivity to a substrate, stereoselectivity to a substrate, and optimal pH value.

31. An apparatus according to claim 20, wherein the amino acid sequence is selected from the group consisting of naturally occurring amino acids, chemically modified amino acids, and non-naturally occurring amino acids.

32. An apparatus according to claim 17 or 19, wherein each member of the multiple-mutated protein population is a molecular complex including at least one protein comprising a plurality of homologous molecules, a plurality of heterologous molecules, or a combination thereof.

33. An apparatus according to claim 17 or 19, further comprising a data storage section.

34. A computer readable recording medium recording a program for executing a method for calculating an optimized solution of the amino acid sequences of multiple-mutated proteins based on input data, the method comprising the steps of:

> searching the three-dimensional structural coordinates of amino acid side chains of the amino acid sequences of members of amultiple-mutated protein population based on the three-dimensional structure data of a template protein population using a dead end elimination algorithm, and executing structural energy minimization calculations for the members, thereby calculating the three-dimensional structural coordinates of an optimum multiple-mutated protein;
> calculating a characteristic value from the three-dimensional structural coordinates of the optimum . multiple-mutated protein; and
> applying a genetic algorithm to the multiple-mutated protein population to calculate the members which optimize the characteristic value.

35. A computer readable recording medium recording a program for executing a method for calculating an optimized solution of the amino acid sequences of multiple-mutated proteins based on input data, the method comprising the steps of:

> (a) inputting sequence data and three-dimensional structure data of a template protein population;
> (b) calculating a characteristic value of each member in the template protein population based on the sequence data and the three-dimensional structure data of the template protein population;

(c) inputting calculation parameters and a desired characteristic value to be used in the algorithm;

(d) applying a genetic algorithm to the template protein population to generate a multiple-mutated protein population based on the calculation parameters, the desired characteristic value and the three-dimensional structure data and the characteristic value of each member in the template protein population;

(e) applying a dead end elimination algorithm to amino acid side chains of amino acid residues of each member in the multiple-mutated protein population to optimize the conformations of the amino acid side chains, and carrying out energy minimization calculations;

(f) calculating three-dimensional structure data and characteristic value of each member having a minimized energy in the multiple-mutated protein population;

(g) determining whether or not steps (h) to (j) are to be carried out based on the calculation parameters, the desired characteristic value, the three-dimensional structure data and the characteristic value of each member in the template protein population, and the three-dimensional structure data and the characteristic value of each member in the multiple-mutated protein population;

(h) when in step (g) it is determined that steps (h) to (j) are carried out, applying a genetic algorithm to the template protein population to generate a new multiple-mutated protein population based on the calculation parameters, the desired characteristic value and the characteristic value of the template protein population, and the characteristic value of each member in the multiple-mutated protein populations which have been generated;

(i) applying the dead end elimination algorithm to amino acid side chains of amino acid residues of each member in the new multiple-mutated protein population to optimize the conformations of the amino acid side chains, and carrying out energy minimization calculations;

(j) calculating three-dimensional structure data and a characteristic value of each member having a minimized energy in the new multiple-mutated protein population;

(k) determining whether or not steps (h) to (j) are carried out based on the calculation parameters, the desired characteristic value, the characteristic value of the template protein population, and the characteristic value of each member in all of the multiple-mutated protein populations which have been generated;

(l) selecting a member having the desired characteristic value from the characteristic values of the members in the template protein population and the characteristic values of the members in all of the multiple-mutated protein populations which have been generated; and

(m) outputting the sequence data and the characteristic value of the selected member.

36. A transmission medium for transmitting a program for executing a method for calculating an optimized solution of the amino acid sequences of multiple-mutated proteins based on input data, the method comprising the steps of:

searching the three-dimensional structural coordinates of amino acid side chains of the amino acid sequences of members of a multiple-mutated protein population based on the three-dimensional structure data of a template protein population using a dead end elimination algorithm, and executing structural energy minimization calculations for the members, thereby calculating the three-dimensional structural coordinates of an optimum multiple-mutated protein;

calculating a characteristic value from the three-dimensional structural coordinates of the optimum multiple-mutated protein; and

applying a genetic algorithm to the multiple-mutated protein population to calculate the members which optimize the characteristic value.

37. A transmission medium for transmitting a program for executing a method for calculating an optimized solution of the amino acid sequences of multiple-mutated proteins based on input data, the method comprising the steps of:

(a) inputting sequence data and three-dimensional structure data of a template protein population;

(b) calculating a characteristic value of each member in the template protein population based on the sequence data and the three-dimensional structure data of the template protein population;

(c) inputting calculation parameters and a desired characteristic value to be used in the algorithm;

(d) applying a genetic algorithm to the template protein population to generate a multiple-mutated protein population based on the calculation parameters, the desired characteristic value and the three-dimensional structure data and the characteristic value of each member in the template protein population;

(e) applying a dead end elimination algorithm to amino acid side chains of amino acid residues of each member in the multiple-mutated protein population to optimize the conformations of the amino acid side chains, and carrying out energy minimization calculations;

(f) calculating three-dimensional structure data and characteristic value of each member having a minimized energy in the multiple-mutated protein population;

(g) determining whether or not steps (h) to (j) are to be carried out based on the calculation parameters, the desired characteristic value, the three-dimensional structure data and the characteristic value of each member in the template protein population, and the three-dimensional structure data and the characteristic value of each member in the multiple-mutated protein population;

(h) when in step (g) it is determined that steps (h) to (j) are carried out, applying a genetic algorithm to the template protein population to generate a new multiple-mutated protein population based on the calculation parameters, the desired characteristic value and the characteristic value of the template protein population, and the characteristic value of each member in the multiple-mutated protein populations which have been generated;

(i) applying the dead end elimination algorithm to amino acid side chains of amino acid residues of each member in the new multiple-mutated protein population to optimize the conformations of the amino acid side chains, and carrying out energy minimization calculations;

(j) calculating three-dimensional structure data and a characteristic value of each member having a minimized energy in the new multiple-mutated protein population;

(k) determining whether or not steps (h) to (j) are carried out based on the calculation parameters, the desired characteristic value, the characteristic value of the template protein population, and the characteristic value of each member in all of the multiple-mutated protein populations which have been generated;

(l) selecting a member having the desired characteristic value from the characteristic values of the members in the template protein population and the characteristic values of the members in all of the multiple-mutated protein populations which have been generated; and

(m) outputting the sequence data and the characteristic value of the selected member.

38. A program for executing a method for calculating an optimized solution of the amino acid sequences of multiple-mutated proteins based on input data, the program causing the computer to execute the processes of:

searching the three-dimensional structural coordinates of amino acid side chains of the amino acid sequences of members of amultiple-mutated protein population based on the three-dimensional structure data of a template protein population using a dead end elimination algorithm, and executing structural energy minimization calculations for the members, thereby calculating the three-dimensional structural coordinates of an optimum multiple-mutated protein;

calculating a characteristic value from the three-dimensional structural coordinates of the optimum multiple-mutated protein; and

applying a genetic algorithm to the multiple-mutated protein population to calculate the members which optimize the characteristic value.

39. A program for executing a method for calculating an optimized solution of the amino acid sequences of multiple-mutated proteins based on input data, the program causing the computer to execute the processes of:

(a) inputting sequence data and three-dimensional structure data of a template protein population, and thereafter, calculating a characteristic value of each member in the template protein population based on the sequence data and the three-dimensional structure data of the template protein population;

(b) inputting calculation parameters and a desired characteristic value to be used in the algorithm, and thereafter, applying a genetic algorithm to the template protein population to generate a multiple-mutated protein population based on the calculation parameters, the desired characteristic value and the three-dimensional structure data and the characteristic value of each member in the template protein population;

(c) applying a dead end elimination algorithm to amino acid side chains of amino acid residues of each member in the multiple-mutated protein population to optimize the conformations of the amino acid side chains, and carrying out energy minimization calculations;

(d) calculating three-dimensional structure data and characteristic value of each member having a minimized energy in the multiple-mutated protein population;

(e) determining whether or not steps (h) to (j) are to be carried out based on the calculation parameters, the desired characteristic value, the three-dimensional structure data and the characteristic value of each member in the template protein population. and the three-dimensional structure data and the characteristic value of each member in the multiple-mutated protein population;

(f) when in step (e) it is determined that steps (h) to (j) are carried out, applying a genetic algorithm to the template protein population to generate a new multiple-mutated protein population based on the calculation parameters, the desired characteristic value and the characteristic value of the template protein population, and the characteristic value of each member in the multiple-mutated protein populations which have been generated;

(g) applying the dead end elimination algorithm to amino acid side chains of amino acid residues of each member in the new multiple-mutated protein population to optimize the conformations of the amino acid side chains, and carrying out energy minimization calculations;

(h) calculating three-dimensional structure data and a characteristic value of each member having a minimized energy in the new multiple-mutated protein population;

(i) determining whether or not steps (f) to (h) are carried out based on the calculation parameters, the desired characteristic value, the characteristic value of the template protein population, and the characteristic value of each member in all of the multiple-mutated protein populations which have been generated;

(j) selecting a member having the desired characteristic value from the characteristic values of the members in the template protein population and the characteristic values of the members in all of the multiple-mutated protein populations which have been generated; and

(k) outputting the sequence data and the characteristic value of the selected member.

**40.** A method for providing a service for calculating an optimized solution of the amino acid sequences of multiple-mutated proteins based on input data over a network, the method comprising:

the step of inputting three-dimensional structure data, amino acid sequence data and calculation parameters of a template protein population to a server, and

the step of the server searching the three-dimensional structural coordinates of amino acid side chains of the amino acid sequences of members of a multiple-mutated protein population based on the three-dimensional structure data of a template protein population using a dead end elimination algorithm, and executing structural energy minimization calculations for the members, thereby calculating the three-dimensional structural coordinates of an optimum multiple-mutated protein;

the step of the server calculating a characteristic value from the three-dimensional structural coordinates of the optimum multiple-mutated protein; and

the step of the server applying a genetic algorithm to the multiple-mutated protein population to calculate the members which optimize the characteristic value.

**41.** A method for providing a service for calculating an optimized solution of the amino acid sequences of multiple-mutated proteins based on input data over a network, the method comprising:

(a) the step of inputting sequence data and three-dimensional structure data of a template protein population;

(b) the step of the server calculating a characteristic value of each member in the template protein population based on the sequence data and the three-dimensional structure data of the template protein population;

(c) the step of inputting calculation parameters and a desired characteristic value to be used in the algorithm;

(d) the step of the server applying a genetic algorithm to the template protein population to generate a multiple-mutated protein population based on the calculation parameters, the desired characteristic value and the three-dimensional structure data and the characteristic value of each member in the template protein population;

(e) the step of the server applying a dead end elimination algorithm to amino acid side chains of amino acid residues of each member in the multiple-mutated protein population to optimize the conformations of the amino acid side chains, and carrying out energy minimization calculations;

(f) the step of the server calculating three-dimensional structure data and characteristic value of each member having a minimized energy in the multiple-mutated protein population;

(g) the step of the server determining whether or not steps (h) to (j) are to be carried out based on the calculation parameters, the desired characteristic value, the three-dimensional structure data and the characteristic value of each member in the template protein population, and the three-dimensional structure data and the characteristic value of each member in the multiple-mutated protein population;

(h) the step of the server, when in step (g) it is determined that steps (h) to (j) are carried out, applying a genetic algorithm to the template protein population to generate a new multiple-mutated protein population based on the calculation parameters, the desired characteristic value and the characteristic value of the template protein population, and the characteristic value of each member in the multiple-mutated protein populations which have been generated;

(i) the step of the server applying the dead end elimination algorithm to amino acid side chains of amino acid residues of each member in the new multiple-mutated protein population to optimize the conformations of the amino acid side chains, and carrying out energy minimization calculations;

(j) the step of the server calculating three-dimensional structure data and a characteristic value of each member having a minimized energy in the new multiple-mutated protein population;

(k) the step of the server determining whether or not steps (h) to (j) are carried out based on the calculation parameters, the desired characteristic value, the characteristic value of the template protein population, and the characteristic value of each member in all of the multiple-mutated protein populations which have been generated;

(l) the step of the server selecting a member having the desired characteristic value from the characteristic values of the members in the template protein population and the characteristic values of the members in all of the multiple-mutated protein populations which have been generated; and

(m) the step of the server outputting the sequence data and the characteristic value of the selected member.

## FIG.1

START

Inputting sequence data and three-dimensional structure data
of a template protein population ⟍10

Calculating a characteristic value of each member in the template
protein population based on the sequence data and the
three-dimensional structure data of the template protein population ⟍12

inputting calculation parameters and a desired characteristic value
to be used in the algorithm ⟍14

Applying a genetic algorithm to the template protein population to
generate a multiple-mutated protein population based on the
calculation parameters, the desired characteristic value and the
three-dimensional structure data and the characteristic value of
each member in the template protein population ⟍20

Applying a dead end elimination algorithm to amino acid side chains of
amino acid residues of each member in the multiple-mutated protein
population to optimize the positions of the amino acid side chains, and
carrying out energy minimization calculations ⟍22

Calculating three-dimensional structure data and characteristic value of each
member having a minimized energy in the multiple-mutated protein population

⟍24

Executing steps 21, 23 and 25? ⟍30        NO

YES                                        ⟋21

Applying a genetic algorithm to the template protein population to generate
a new multiple-mutated protein population based on the calculation
parameters, the desired characteristic value and the characteristic value of
the template protein population, and the characteristic value of each member
in the multiple-mutated protein populations which have been generated

Applying the dead end elimination algorithm to amino acid side chains of
amino acid residues of each member in the new multiple-mutated protein
population to optimize the positions of the amino acid side chains, and
carrying out energy minimization calculations ⟍23

calculating three-dimensional structure data and a characteristic value of
each member having a minimized energy in the new multiple-mutated protein
population;

⟍25

YES        Executing steps 21, 23 and 25 again? ⟋31

NO

Selecting a member having the desired characteristic value from the
characteristic value of each member in the template protein population and
the characteristic value of each member in all of the multiple-mutated
protein populations which have been generated

⟍40

Outputting the sequence data and the characteristic value of the
selected member

⟍50

END

*FIG.2*

Current generation
multiple-mutated protein
amino acid sequence population —201

Selection based on the
characteristic values and
selection rates of proteins —202

Current generation
multiple-mutated protein
characteristic database —203

Reproduction based on a change in
the number of individuals and
the reproduction rate of the
population —204

Crossover based on a crossover rate —206

Mutation based on a mutation rate —208

Next generation
multiple-mutated protein
amino acid sequence population —210

*FIG.3*

EP 1 241 598 A1

```
┌─────────────────────┐  ─201
│ Current generation  │
│ multiple-mutated    │
│ protein             │
│ amino acid sequence │
│ population          │
└─────────────────────┘
          │
          │                  ─220
          ▼
┌─────────────────┐
│ The amino acid  │──────────────────►
│ sequence of each│
│ multiple-mutated│
│ protein         │
└─────────────────┘
```

```
                        ┌──────────────────────┐ ─101
                        │ Template protein     │
                        │ atomic coordinates   │
                        └──────────────────────┘
                                   │
                                   ▼                        ─222
┌──────────────────────────────────────────────────────┐
│ Superimposing the amino acid atomic                    │
│ coordinates of each mutated protein onto               │
│ the higher-order structure of the template             │
│ protein                                                │
└──────────────────────────────────────────────────────┘
                                   │
                                   ▼                        ─224
┌──────────────────────────────────────────────────────┐
│ Partially optimization of the atomic                   │
│ coordinates of the amino acid side chains              │
│ of each mutated protein by a dead end                  │
│ elimination algorithm                                  │
└──────────────────────────────────────────────────────┘
```

```
          ─228
┌─────────────────┐
│ The atomic      │◄───────────
│ coordinates of  │
│ each            │
│ multiple-mutated│
│ protein         │
└─────────────────┘
```

```
                                                  ─226
┌──────────────────────────────────────────────────────┐
│ Global optimization of the atomic                      │
│ coordinates of each mutated protein by                 │
│ energy minimization calculation                        │
└──────────────────────────────────────────────────────┘
```

```
          ─230
┌─────────────────┐
│ Current generation│
│ multiple-mutated │
│ protein          │
│ atomic coordinates│
│ population       │
└─────────────────┘
          │
          ▼
┌──────────────────────┐                    ─242
│ Calculation of a     │        ─240    ┌──────────────────────┐
│ characteristic value │                │ Next generation      │
│ of each mutated      │───────────────►│ multiple-mutated     │
│ protein based on the │                │ protein              │
│ atomic coordinates   │                │ characteristic value │
│ of the protein       │                │ data base            │
└──────────────────────┘                └──────────────────────┘
```

## FIG.4

```
                    ┌─────────────────────────────┐
                    │   Reading of input file     │
                    └─────────────────────────────┘
                                  ↓
        ┌──────────────────────────────────────────────┐
        │  Preparation of amino acid residue rotamers   │
        └──────────────────────────────────────────────┘
                                  ↓
   ┌───────────────────────────────────────────┐      ┌──────────────────────┐
   │ Calculation of energies in amino acid     │      │ Intra/inter-residue  │
   │ residues and between amino acid residues · │- - ->│ energy stored        │
   │ Deletion of rotamers greater than or      │      │ data                 │
   │ equal to threshold                        │      └──────────────────────┘
   └───────────────────────────────────────────┘
                                  ↓
   ┌───────────────────────────────────────────────┐
   │ Production of a first-(next-) generation       │
   │ mutated amino acid sequence colony             │
   └───────────────────────────────────────────────┘
                                  ↓
     ┌─────────────────────────────────────────────┐
     │ Production of a next-generation mutated      │
     │ amino acid sequence colony                   │
     └─────────────────────────────────────────────┘
                                  ↓
     ┌─────────────────────────────────────────────┐
     │ Selection of amino acid sequence individuals │
     └─────────────────────────────────────────────┘
                                  ↓
     ┌───────────────────────────────────────────────┐
     │ Reproduction of amino acid sequence individuals│
     └───────────────────────────────────────────────┘
                                  ↓
     ┌─────────────────────────────────────────────┐
     │ Crossover of amino acid sequence individuals │
     └─────────────────────────────────────────────┘
                                  ↓
     ┌─────────────────────────────────────────────┐
     │ Mutation of amino acid sequence individuals  │
     └─────────────────────────────────────────────┘
                                  ↓
     ┌─────────────────────────────────────────────┐
     │ Storage of a current-generation mutated      │
     │ amino acid sequence colony                   │
     └─────────────────────────────────────────────┘
                                  ↓
     ┌─────────────────────────────────────────────────┐
     │ Production of a (next) mutated amino acid        │
     │ sequence                                         │
     └─────────────────────────────────────────────────┘
                                  ↓
  YES  ┌─────────────────────────────────────────────┐
 <─────│ Is the sequence already subjected to dead end│
       │ elimination?                                 │
       └─────────────────────────────────────────────┘
                                  ↓
       ┌──────────────────────────────────────────────────┐
       │ Dead end elimination calculation                 │
       │ · Interaction between rotamers                   │
       │ · Interaction between combinations of rotamers   │
       └──────────────────────────────────────────────────┘
                                  ↓
            ┌──────────────────────────────────────┐
            │ Energy minimization calculation       │
            └──────────────────────────────────────┘
                                  ↓
            ┌──────────────────────────────────────┐      ┌──────────────┐
            │ Structural energy calculation         │- - ->│ Calculation  │
            └──────────────────────────────────────┘      │ results      │
                                  ↓                        │ stored data  │
     ┌─────────────────────────────────────────────┐      └──────────────┘
     │ Are all mutated amino acid sequenced in the  │
 NO  │ colony already subjected to the calculations?│
     └─────────────────────────────────────────────┘
                            YES  ↓
                    ┌─────────────────────┐
                    │  Final generation?  │
                    └─────────────────────┘
                            NO ↓
   ┌───────────────────────────────────────────────────────────┐
 NO│ Are all colonies already subjected to the calculations?    │
   └───────────────────────────────────────────────────────────┘
                            YES ↓
              ┌─────────────────────────────────┐
              │  Production of an output file    │ <- - - -
              └─────────────────────────────────┘
```

33

## FIG.5A

| Rank | Residue-36 | Residue-40 | Residue-47 | Difference in structural energy value (kcal/mol) |
|---|---|---|---|---|
| 1 | V | V |  | 0.00 |
| 2 |  | A |  | -0.77 |
| 3 | L | L | A | -1.36 |
| 4 | V | L | A | -1.91 |
| 5 | V | M | I | -2.20 |
| 6 |  | M |  | -2.31 |
| 7 | L | L | A | -2.63 |
| 8 | V | L | M | -2.65 |
| 9 | V | V | V | -2.73 |
| 10 | V | L | M | -2.87 |
| 11 | M | M | A | -2.91 |
| 12 | V | L | L | -2.97 |
| 13 | V | M | E | -3.08 |
| 14 | V | M | M | -3.18 |
| 15 | V | L | E | -3.24 |
| 16 | V | L | M | -3.25 |
| 17 | V | L | E | -3.28 |
| 18 | V | M |  | -3.28 |
| 19 | V | M | A | -3.46 |
| 20 | V | V | A | -3.50 |
| 21 | V | M | A | -3.53 |
| 22 | V | A | M | -3.69 |
| 23 | V | A | E | -3.72 |
| 24 | E | M | E | -3.73 |
| 25 | M | K | S | -3.86 |
| 26 | M | M | A | -3.97 |
| 27 | M | M | M | -4.01 |
| 28 | L | M | E | -4.01 |
| 29 | L | N | E | -4.29 |
| 30 | V | S | A | -4.32 |
| 31 | V | M | L | -4.41 |
| 32 | V | V | A | -4.43 |
| 33 | A | M | E | -4.46 |
| 34 | M | V | E | -4.52 |
| 35 | V | V | M | -4.54 |
| 36 | A | M | E | -4.58 |
| 37 | A | A | M | -4.63 |
| 38 | V | A | E | -4.64 |
| 39 | V | A | A | -4.64 |
| 40 | V | M | E | -4.65 |
| 41 | V | M | E | -4.67 |
| 42 | V | K | E | -4.72 |
| 43 | L | M | V | -4.77 |
| 44 |  | V | E | -4.81 |
| 45 |  | V | L | -4.84 |
| 46 | V | A | L | -4.88 |
| 47 | V | A | E | -4.88 |
| 48 | V | L | E | -4.89 |
| 49 | M | L | E | -4.89 |
| 50 | V | L | E | -4.91 |
| 51 | V | A | E | -4.91 |
| 52 | V | V | T | -4.91 |
| 53 | V | F | A | -4.92 |
| 54 | V | S | A | -4.99 |
| 55 | M | F | E | -4.99 |
| 56 | V | N | M | -5.02 |
| 57 | M | A | M | -5.03 |
| 58 | M | V | M | -5.10 |
| 59 | V | L | S | -5.10 |
| 60 | V | L | S | -5.10 |

# FIG.5B

| | | | | | |
|---|---|---|---|---|---|
| 61 | V | V | L | | −5.12 |
| 62 | | F | | | −5.12 |
| 63 | D | M | | | −5.13 |
| 64 | M | F | M | | −5.18 |
| 65 | A | L | V | | −5.21 |
| 66 | V | V | | | −5.22 |
| 67 | | A | E | | −5.31 |
| 68 | | S | A | | −5.31 |
| 69 | | F | I | | −5.32 |
| 70 | | N | A | | −5.33 |
| 71 | | H | V | | −5.34 |
| 72 | | A | | | −5.34 |
| 73 | A | | A | | −5.36 |
| 74 | | F | | | −5.36 |
| 75 | E | | A | | −5.37 |
| 76 | M | V | A | | −5.39 |
| 77 | I | | M | | −5.48 |
| 78 | | | | | −5.53 |
| 79 | V | M | M | | −5.54 |
| 80 | | V | M | | −5.54 |
| 81 | I | | M | | −5.60 |
| 82 | | | E | | −5.61 |
| 83 | | | | | −5.63 |
| 84 | V | F | S | | −5.63 |
| 85 | V | | E | | −5.67 |
| 86 | A | V | V | | −5.68 |
| 87 | A | V | | | −5.70 |
| 88 | | L | | | −5.71 |
| 89 | | | V | | −5.74 |
| 90 | V | | | | −5.78 |
| 91 | | V | M | | −5.81 |
| 92 | A | L | A | | −5.82 |
| 93 | L | | A | | −5.83 |
| 94 | | L | S | | −5.87 |
| 95 | | | V | | −5.88 |
| 96 | | F | L | | −5.89 |
| 97 | D | L | L | | −5.93 |
| 98 | A | L | M | | −5.99 |
| 99 | E | M | A | | −5.99 |
| 100 | M | | M | | −6.02 |
| 101 | M | L | | | −6.02 |
| 102 | | M | | | −6.10 |
| 103 | V | | A | | −6.10 |
| 104 | | L | D | | −6.12 |
| 105 | M | M | A | | −6.13 |
| 106 | V | M | M | | −6.13 |
| 107 | V | M | | | −6.16 |
| 108 | | | | | −6.16 |
| 109 | M | M | A | | −6.16 |
| 110 | M | M | L | | −6.18 |
| 111 | E | L | A | | −6.18 |
| 112 | L | V | S | | −6.18 |
| 113 | | | S | | −6.20 |
| 114 | | L | E | | −6.21 |
| 115 | V | | E | | −6.28 |
| 116 | A | | V | | −6.28 |
| 117 | A | M | V | | −6.34 |
| 118 | | L | | | −6.35 |
| 119 | | V | | | −6.40 |
| 120 | M | V | L | | −6.42 |

# FIG.6

<table>
<tr><td colspan="2"><b>INTERNATIONAL SEARCH REPORT</b></td><td>International application No.<br>PCT/JP00/09127</td></tr>
</table>

| | |
|---|---|
| A. CLASSIFICATION OF SUBJECT MATTER<br>    Int.Cl⁷   G06F17/50 | |

$Int.Cl^7$   G06F17/50

According to International Patent Classification (IPC) or to both national classification and IPC

**B. FIELDS SEARCHED**

Minimum documentation searched (classification system followed by classification symbols)
    $Int.Cl^7$   G06F17/50, G06F17/30, G06N3/12

Documentation searched other than minimum documentation to the extent that such documents are included in the fields searched

Electronic data base consulted during the international search (name of data base and, where practicable, search terms used)
    JICST, JQUICK FILE(JOIS)

**C. DOCUMENTS CONSIDERED TO BE RELEVANT**

| Category* | Citation of document, with indication, where appropriate, of the relevant passages | Relevant to claim No. |
|---|---|---|
| Y | Soichi MORIKAWA, et al., "DEE wo Riyou shita Tanpakushitsu Heni  Sekkei Program : Shrike", Nippon Seibutsu Butsuri Gakkai Dai 37 kai Nenkai Kouen Yokoushuu,  page S114, 02 September, 1999,  Full text | 1-41 |
| Y | J. R. Desjarlais et al., "Side-chain and Backbone Flexibility in Protein Core Design", Journal of Molecular Biology, Vol. 290, No. 1, 02 July 1999, Page 306, right column, line 41 to page 307, right column, line 5, "Program Description" | 1-41 |
| A | Ryuuji TANIMURA, et al., "Tanpakushitsu no Modeling to Sekkei", CICSJ Bulletin, Vol. 11, No. 4, pp. 37-40, Full text | 1-41 |

| ☐ Further documents are listed in the continuation of Box C. | ☐ See patent family annex. |
|---|---|

| * Special categories of cited documents:<br>"A"  document defining the general state of the art which is not considered to be of particular relevance<br>"E"  earlier document but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |
|---|---|
| Date of the actual completion of the international search<br>    02 March, 2001 (02.03.01) | Date of mailing of the international search report<br>    13 March, 2001 (13.03.01) |
| Name and mailing address of the ISA/<br>    Japanese Patent Office | Authorized officer |
| Facsimile No. | Telephone No. |

Form PCT/ISA/210 (second sheet) (July 1992)